# EUROPEAN PATENT SPECIFICATION

(11) **EP 4 041 157 B1**
(45) Date of publication and mention of the grant of the patent: **27.11.2024**
(21) Application number: 20792739.3
(22) Date of filing: 06.10.2020
(51) Int. Cl.: A61F 13/05, A61F 13/14, A61M 1/00

(54) **DRESSING FOR BREAST CAVITIES**
VERBAND FÜR BRUSTKAVITÄTEN
PANSEMENT POUR CAVITÉS MAMMAIRES

(30) Priority: 08.10.2019 US 201962912265 P
(43) Date of publication of application: 17.08.2022
(73) Proprietor: Solventum Intellectual Properties Company, Maplewood, MN 55144 (US)
(72) Inventor: LOCKE, Christopher Brian, San Antonio, Texas 78265 (US); ROBINSON, Timothy Mark, San Antonio, Texas 78265 (US); EDWARDS, Thomas, San Antonio, Texas 78265 (US); INGRAM, Shannon C., San Antonio, Texas 78265 (US)
(74) Representative: Simmons & Simmons
(86) International application number: PCT/IB2020/059373
(87) International publication number: WO 2021/070051

(56) References cited:
- EP-A1- 3 406 275
- WO-A1-2019/084006
- US-A1- 2016 199 550
- US-A1- 2018 214 315

## Description

### CROSS-REFERENCE TO RELATED APPLICATIONS

This application claims the benefit of priority to U.S. Provisional Application No. 62/912,265, filed on October 8, 2019.

### BACKGROUND

The present invention relates generally to the field of wound dressings, and more particularly to wound dressings for breast cavities.

Breast implants can become infected due to a variety of reasons. In particular, after malignant tumor removal in a breast, an implant made be surgically placed within the breast cavity. If such an implant becomes infected, these infections may be localized in the tissue surrounding the implant or may be systemic (e.g., affecting the entire body rather than the localized tissue surrounding the implant). The infection rate may be higher for patients who had breast cancer and have had a mastectomy to reconstruct the breast. However, infection can occur even if the implants are placed for cosmetic reasons. It would be desirable to provide a dressing for treating infections in a body cavity such as a post-surgical breast cavity.

US2016/199550 and US2018/214315 disclose wound dressings.

### SUMMARY

One implementation of the present invention is a dressing for an internal cavity as claimed in claim 1. The dressing includes a connector, a negative pressure pathway layer, an instillation pathway layer, a negative pressure manifold, and an instillation manifold. The negative pressure pathway layer has a proximate end coupled to the connector and a distal end that is substantially free. The instillation pathway layer has a proximate end coupled with the connector and a distal end that is substantially free. The negative pressure manifold is disposed within the negative pressure pathway layer. A proximate end of the negative pressure manifold is fluidly coupled with a first channel of the connector. The instillation manifold is disposed within the instillation pathway layer. The proximate end of the instillation manifold is fluidly coupled with a second channel of the connector. The negative pressure pathway layer and the instillation pathway layer are configured to cooperatively form an inner volume therebetween. The inner volume is configured to receive a space filler. The negative pressure pathway layer, the instillation pathway layer, and the space filler are collectively configured to be positioned within the internal cavity.

### BRIEF DESCRIPTION OF THE DRAWINGS

Various objects, aspects, features, and advantages of the disclosure will become more apparent and better understood by referring to the detailed description taken in conjunction with the accompanying drawings, in which like reference characters identify corresponding elements throughout. In the drawings, like reference numbers generally indicate identical, functionally similar, and/or structurally similar elements.
FIG. 1 is a side view of a clamshell dressing for a breast cavity including an instillation pathway layer and a negative pressure pathway layer, according to some embodiments.
FIG. 2 is a side view of the dressing of FIG. 1 with a space filler positioned between the negative pressure pathway layer and the instillation pathway layer, according to some embodiments.
FIG. 3 is a top view of the dressing of FIG. 1 including radio-opaque strips, according to some embodiments.
FIG. 4 is a detailed view of the negative pressure pathway layer including an inner and outer film layer and a manifold structure, according to some embodiments.
FIG. 5 is a diagram of a connector of the dressing of FIG. 1 in a first position, according to some embodiments.
FIG. 6 is a diagram of the connector of the dressing of FIG. 1 in a second position, according to some embodiments.
FIG. 7 is a bottom view of the dressing of FIG. 1, according to some embodiments.
FIG. 8 is a side view of the dressing of FIG. 1, showing a flow of instillation fluid into the breast cavity and a flow of removed fluid from the breast cavity, according to some embodiments.
FIG. 9 is a side view of the dressing of FIG. 1 implanted in a patient's breast cavity, according to some embodiments.
FIG. 10 is a side view of an inflatable dressing, including a negative pressure pathway layer and an instillation pathway layer that cooperatively define a sealed inner volume in a deflated state, according to some embodiments.
FIG. 11 is a side view of the dressing of FIG. 10 in an inflated state, according to some embodiments.
FIG. 12 is a side view of the dressing of FIG. 10 showing a flow of instillation fluid into the breast cavity and a flow of fluid out of the breast cavity, according to some embodiments.
FIG. 13 is a side view of the dressing of FIG. 10 implanted in a patient's breast cavity, according to some embodiments.
FIG. 14 is a diagram of a system for providing negative pressure therapy to a patient's breast cavity with a permanent implant, according to some embodiments.
FIG. 15 is a block diagram of a negative pressure wound therapy system including a therapy unit coupled with a wound or a cavity via tubing, according to some embodiments.
FIG. 16 is a block diagram illustrating the therapy unit of FIG. 15 in greater detail when the therapy unit operates to draw a vacuum within a negative pressure circuit, according to some embodiments.
FIG. 17 is a block diagram illustrating the therapy unit of FIG. 15 in greater detail when the therapy unit operates to vent the negative pressure circuit, according to some embodiments.
FIG. 18 is a block diagram illustrating the therapy unit of FIG. 15 in greater detail when the therapy unit uses an orifice to vent the negative pressure circuit, according to some embodiments.
FIG. 19 is a block diagram illustrating the therapy unit of FIG. 15 in greater detail when the therapy unit operates to deliver instillation fluid to the wound dressing and/or a wound and/or a cavity, according to some embodiments.
FIG. 20 is a flow diagram of a process for using the dressing of FIGS. 1-9, according to some embodiments.
FIG. 21 is a flow diagram of a process for using the dressing of FIGS. 10-13, according to some embodiments.
FIG. 22 is a flow diagram of a process for using the dressing of FIG. 14, according to some embodiments.
FIG. 23 is a diagram of a temporary breast implant, according to some embodiments.

### DETAILED DESCRIPTION

### Overview

Referring generally to the FIGURES, various negative pressure dressings and systems for breast cavities are shown. The dressing can include a negative pressure pathway layer and an instillation pathway layer. The negative pressure pathway layer and the instillation pathway layer each include a manifold that is configured to absorb, transport, wick, etc., fluid therethrough. The negative pressure pathway layer includes a negative pressure manifold, an outer film layer and an inner film layer. The instillation pathway layer includes an instillation manifold, an outer film layer and an inner film layer. The outer film layers and/or the inner film layers of the negative pressure pathway layer and the instillation pathway layer can include holes, openings, perforations, fenestrations, apertures, etc., to allow or facilitate the transfer of fluid therethrough.

The negative pressure pathway layer and the instillation pathway layer can be fixedly and/or fluidly coupled at a first end with a connector. The negative pressure pathway layer and the instillation pathway layer may also be sealingly coupled with each other at a second or distal end with each other. In a first embodiment, the negative pressure pathway layer and the instillation pathway layer are not sealingly coupled with each other at the distal or second end, but rather, are free at the second or distal end. In a second embodiment, the negative pressure pathway layer and the instillation pathway layer are sealingly coupled (e.g., welded using a radio frequency/high frequency ("RF/HF") weld) so that the negative pressure pathway layer and the instillation pathway layer cooperatively define a sealed inner volume.

The connector can include various channels that are configured to facilitate the transport of fluid to or from the manifolds of the negative pressure pathway layer and the instillation pathway layer. For example, a first channel may fluidly couple with the negative pressure manifold so that fluid absorbed, wicked, transferred, etc., through the negative pressure manifold can be transferred through the first channel of the connector. Likewise, a second channel may extend through the connector and fluidly couple with the instillation manifold. Instillation fluid can be provided through the second channel to the instillation manifold. The instillation manifold may absorb, wick, transfer, transport, etc., the instillation fluid. The instillation fluid may exit from the instillation manifold through the openings, apertures, perforations, fenestrations, etc., in the inner and/or outer layer of the instillation pathway layer.

The dressing can be positioned within a patient's breast cavity. If the second or distal ends of the instillation pathway layer and the negative pressure pathway layer are free, the instillation pathway layer and the negative pressure pathway layer may be wrapped around a space filler (e.g., a breast expander, a temporary implant, etc.). If the second or distal ends of the instillation pathway layer and the negative pressure pathway layer are sealed or welded with each other, the dressing may have the form of an inflatable bladder. The dressing can be implanted into the patient's breast cavity by a medical professional. After the dressing is implanted into the patient's breast cavity, the dressing may be inflated (e.g., if the second or distal ends of the instillation pathway layer and the negative pressure pathway layer are sealed or welded with each other). The sealed inner volume of the dressing may be fluidly coupled with a third or central or medial channel of the connector. A fluid delivery device can be removably fluidly coupled with the third channel of the connector and may be operated to introduce fluid (e.g., liquid such as a saline solution, gas, air, etc.) to the sealed inner volume of the dressing. The fluid delivery device can be a syringe or a pump, or any other device capable of delivering fluid to the sealed inner volume of the dressing. The dressing can be inflated until the dressing reaches a desired size.

The connector may be fluidly coupled with a vacuum tube and an instillation tube. The vacuum tube can be configured to fluidly couple (e.g., removably) with the first channel of the connector. The instillation tube can be configured to fluidly couple (e.g., removably) with the second channel of the connector. The instillation tube and the vacuum tube can be fluidly coupled with a therapy unit that is configured to both draw a negative pressure at the patient's breast cavity through the vacuum tube as well as to deliver instillation fluid to the breast cavity through the instillation tube. The therapy unit may include a pneumatic pump that is configured to draw a negative pressure and wound fluid from/at the patient's breast cavity through the vacuum tube and the negative pressure pathway layer. The therapy unit can also include an instillation pump that is configured to deliver or drive the instillation fluid to the patient's breast cavity through the instillation tube and the instillation pathway layer.

In other embodiments, the vacuum tube, the instillation tube, and the therapy unit are configured for use with a permanent breast implant. For example, the instillation tube can be surgically inserted so that an open end of the instillation tube fluidly couples with an upper portion of the patient's breast cavity. In this way, instillation fluid can be delivered to the patient's breast cavity at the upper portion. The breast cavity can include a permanent breast implant that is positioned therewithin. The instillation fluid can be provided to the patient's breast cavity at the upper portion, and may flow through a space defined between the permanent breast implant and an interior facing surface of the patient's breast cavity. The vacuum tube can be surgically inserted and fluidly coupled at a bottom or lower portion of the patient's breast cavity. The vacuum tube can be configured to draw a negative pressure within the patient's breast cavity. The vacuum tube can also be configured to draw or remove fluid from the patient's breast cavity (e.g., exuded wound fluid, instillation fluid, etc.). In this way, instillation fluid can be provided to the patient's breast cavity at the upper portion, may flow downwards to the lower portion, and be removed from the patient's breast cavity through the vacuum tube.

Breast implants may become infected, for example, after malignant tumor removal in the breast. In some cases, permanent implants can become infected after they have been surgically inserted. This may occur weeks or even months after the implant is placed. Other approaches to treating implant infection include removing the breast implant, treating the infection with the implant via systemic antibiotics, or the use of drains to administer localized washes and antibiotics to the implant site. However, in most cases, the implant must be removed, the capsule thoroughly washed, etc., so that the infection can be controlled. Such procedures may take 3-6 months and can be uncomfortable for patients. Additionally, removing the infected implant can be distressing to the patient, since the patient's breasts may become unsymmetrical. Advantageously, the systems and methods described herein facilitate maintaining an implant in the breast cavity, while administering negative pressure therapy to control the infection. Advantageously, this facilitates a less invasive approach to treating infections in bodily cavities (e.g., breast cavities) while providing symmetrical appearances and thereby reducing patient distress.

### Clamshell Dressing

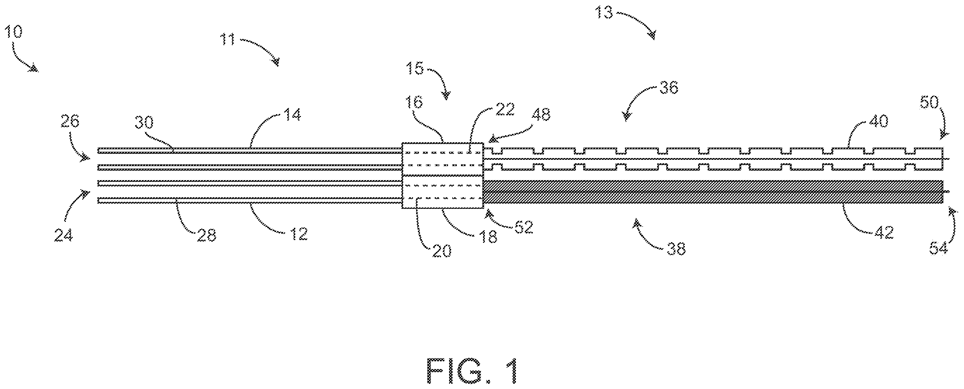

Referring to FIG. 1, a dressing assembly 10 for a breast cavity or other internal bodily cavity is shown. Dressing assembly 10 includes a dressing 13 and a connection assembly 11. Connection assembly 11 includes a connector 15, and several conduits, tubular members, pipes, hoses, tubes, etc., shown as instillation tube 12 and vacuum tube 14. Connector 15 includes a first portion, a vacuum portion, a negative pressure portion, etc., shown as vacuum portion 16, and a second portion, an instillation portion, etc., shown as instillation portion 18. Vacuum portion 16 includes a first channel, a first bore, a through-hole, a fluid passageway, a fluid path, an opening, an inner volume, an aperture, etc., shown as vacuum channel 22. Instillation portion 18 includes a second channel, a second bore, a through-hole, a fluid passageway, a fluid path, an opening, an inner volume, an aperture, etc., shown as instillation channel 20. Instillation channel 20 may extend through instillation portion 18 of connector 15, while vacuum channel 22 extends through vacuum portion 16 of connector 15.

Instillation tube 12 can include an inner volume 28, and an inlet end 24. An outlet end of instillation tube 12 is the opposite end of instillation tube 12 that may be fluidly coupled with instillation channel 20. Likewise, vacuum tube 14 can include an inner volume 30 and an inlet end 26. An outlet end of vacuum tube 14 is the opposite end of vacuum tube 14 that may be fluidly coupled with vacuum channel 22.

Dressing 13 includes a manifold portion, a manifold member, etc., shown as negative pressure pathway layer 36, and an instillation portion, an instillation member, etc., shown as instillation pathway layer 38. Negative pressure pathway layer 36 and instillation pathway layer 38 can be elongated members that couple (e.g., fixedly, fluidly, etc.) at one end with connector 15, and are free at an opposite end. Negative pressure pathway layer 36 may be coupled (e.g., fixedly) with connector 15 at proximate end 48, while distal end 50 of negative pressure pathway layer 36 is free. Likewise, instillation pathway layer 38 can be coupled (e.g., fixedly) with connector 15 at proximate end 52, while distal end 54 of instillation pathway layer 38 is free.

Negative pressure pathway layer 36 can include a manifold structure, a foam structure, a gauze structure, a webbed material, a thermally molded structure, etc., shown as negative pressure manifold 40 that is configured to facilitate the absorption of fluid therethrough. Negative pressure manifold 40 can extend outwards from connector 15 and may be fixedly and/or fluidly coupled with connector 15 at proximate end 48. Distal end 50 of negative pressure manifold 40 can be free.

Instillation pathway layer 38 can include a similar manifold structure, a similar foam structure, a gauze structure, a webbed material, a thermally molded structure, etc., shown as instillation manifold 42. Instillation manifold 42 can be made from a material that is the same as or similar to negative pressure manifold 40. In some embodiments, instillation manifold 42 and negative pressure manifold 40 are manufactured from a hydrophilic material. The hydrophilicity of instillation manifold 42 may be greater than the hydrophilicity of negative pressure manifold 40.

Instillation manifold 42 can be fluidly coupled with instillation channel 20. In some embodiments, instillation manifold 42 is received within instillation channel 20. Fluid can be provided to instillation manifold 42 through instillation channel 20. The fluid may be wicked or absorbed through instillation manifold 42 and provided to a body cavity (e.g., a breast cavity) in which dressing assembly 10 or dressing 13 is positioned. The fluid may be driven to absorb, wick, flow, or more generally, be transferred through instillation manifold 42 due to a negative pressure in the body cavity.

Negative pressure manifold 40 can be fluidly coupled with vacuum channel 22. In some embodiments, negative pressure manifold 40 is received within vacuum channel 22. A negative pressure can be drawn within the cavity in which dressing assembly 10 or dressing 13 is positioned through negative pressure manifold 40. Negative pressure manifold 40 can also be configured to wick, absorb, or more generally, transfer fluid (e.g., wound exudate) from the cavity (e.g., from the breast cavity). The fluid can be wicked, absorbed, transferred, or may flow through negative pressure manifold 40 to vacuum channel 22. The fluid can then be drawn into a therapy unit that produces or draws down the negative pressure. In some embodiments, the therapy unit is fluidly coupled with the vacuum channel 22 and the instillation channel 20. The therapy unit can be configured to draw a negative pressure at the cavity while also providing instillation fluid to the cavity.

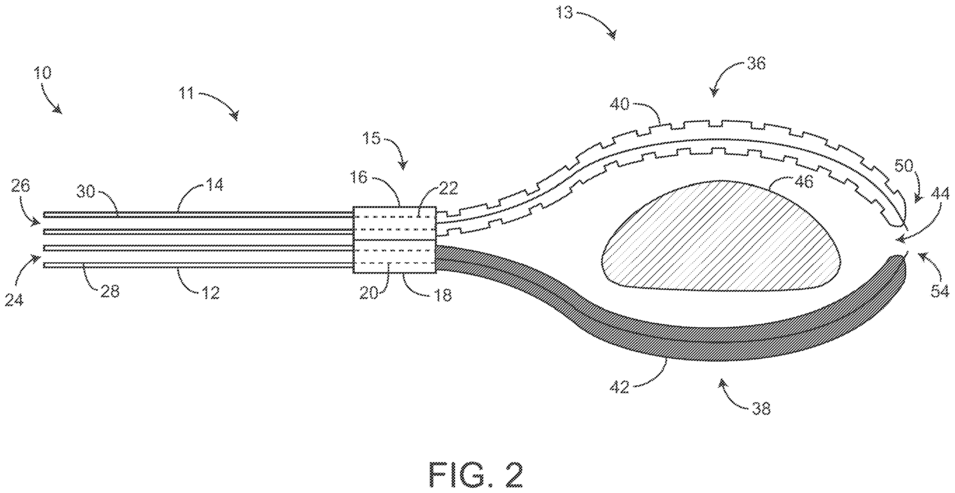

Referring particularly to FIG. 2, the free ends (e.g., distal end 50 and distal end 54) can be adjusted or translated such that an inner volume, a space, an opening, etc., shown as inner volume 44 is formed or defined between negative pressure pathway layer 36 and instillation pathway layer 38. A space filler, an implant, a permanent implant, a temporary implant, etc., shown as space filler 46 can be positioned within inner volume 44, between negative pressure pathway layer 36 and instillation pathway layer 38. Space filler 46 can be a breast expander, an implant, etc., or any other object (e.g., an expandable or inflatable bladder). Instillation pathway layer 38 and negative pressure pathway layer 36 can be wrapped around space filler 46 such that instillation pathway layer 38, negative pressure pathway layer 36, and space filler 46 are positioned within the cavity. An interior or inwards facing surface of negative pressure pathway layer 36 and instillation pathway layer 38 can be configured to directly contact or engage space filler 46. Likewise, an exterior surface of negative pressure pathway layer 36 and instillation pathway layer 38 can be configured to engage or directly contact an interior or inwards facing surface of the cavity.

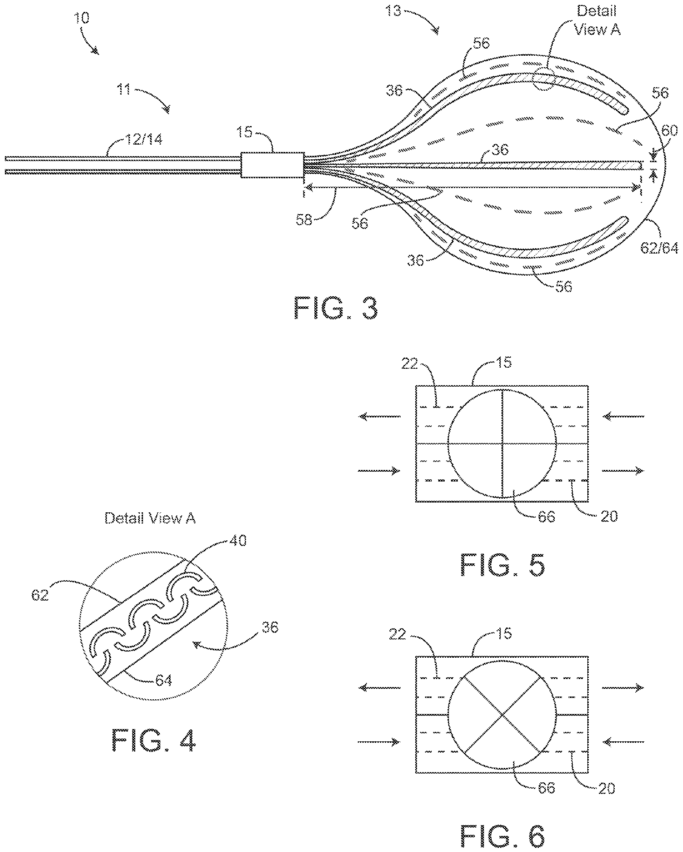

Referring particularly to FIGS. 3 and 4, negative pressure pathway layer 36 and/or instillation pathway layer 38 can have an overall length 58. Negative pressure pathway layer 36 and instillation pathway layer 38 can be elongated members that extend substantially an entire length of dressing 13. Negative pressure pathway layer 36 and instillation pathway layer 38 can each have a width 60. In some embodiments, width 60 is between 10 and 50 millimeters. Negative pressure pathway layer 36 and/or instillation pathway layer 38 can have a thickness of 6 to 10 millimeters.

Dressing 13 can include multiple negative pressure pathway layers 36 and multiple instillation pathway layers 38, or multiple negative pressure manifolds 40 and multiple instillation manifolds 42. Manifolds 40 and 42 may function as legs, webs, fingers, etc., to provide or distribute the instillation fluid (e.g., to provide the instillation fluid to the cavity in multiple locations) and to produce a uniform/distributed negative pressure within the cavity. For example, negative pressure pathway layer 36 and instillation pathway layer 38 can include multiple manifolds 40 and 42 (e.g., open-celled foam strips) to evenly distribute the negative pressure and the instillation fluid. In some embodiments, the film layers 62 and 64 are formed or molded to include channels for the multiple manifolds 40 and 42 to extend through.

Referring particularly to FIG. 4, negative pressure manifold 40 of negative pressure pathway layer 36 can be positioned between a first or an outer or a top film layer 62 and a second or an inner or a bottom film layer 64. Film layers 64 and 62 can be polyurethane layers that are spaced a distance apart. For example, film layers 64 and 62 can be a Vancive MED-9555 film. Negative pressure manifold 40 is sandwiched between film layers 64 and 62 and can extend along film layers 64 and 62 through the space defined therebetween. Negative pressure manifold 40 can be or include a foam webbing, a three-dimensional textile, a web, a gauze, a vacuum formed structure, an embossed structure, etc. For example, negative pressure manifold 40 can be defined between outer film layer 62 and inner film layer 64. Outer film layer 62 and inner film layer 64 can be thermoformed such that one or more channels, vacuum passageways, passages, etc., extend therebetween film layers 62 and 64 and fluidly couple with vacuum channel 22. Instillation pathway layer 38 can be configured similarly to negative pressure pathway layer 36 and may include similar film layers with instillation manifold 42 positioned therebetween. Outer film layer 62 and inner film layer 64 can be polyurethane film layers. In some embodiments, outer film layer 62 and inner film layer 64 have a thickness of approximately 80 micrometers.

In some embodiments, outer film layer 62 includes one or more perforations, fenestrations, openings, etc. The one perforations can be configured to facilitate the egress or entry of fluid between the cavity and the spaced defined between film layers 62 and 64. For example, fluid may flow through the space defined between film layers 62 and 64 of instillation pathway layer 38, and exit the space through the openings of outer film layer 62. Instillation manifold 42 can be positioned between film layers 62 and 64 and may facilitate the transport of instillation fluid through the space between film layers 62 and 64 from instillation channel 20 to various locations within the cavity. Likewise, negative pressure manifold 40 can be positioned between film layers 62 and 64 and may facilitate the transport of fluid from the cavity towards a negative pressure source (e.g., towards a therapy unit, towards connector 15, etc.). Fluid within the cavity (e.g., exuded wound fluid, instillation fluid, etc.) can be transferred to negative pressure manifold 40 through the openings in outer film layer 62. Additionally, the openings in outer film layer 62 can facilitate providing the negative pressure to the cavity.

In some embodiments, outer film layer 62 and inner film layer 64 include thermoformed passageways, structures, etc. For example, outer film layer 62 and inner film layer 64 may be integrally formed with each other but offset a distance apart to define various passageways or fluid flow paths therebetween. If this is the case, outer film layer 62 and inner film layer 64 may be thicker (e.g., 100 micrometers to 250 micrometers). Outer film layer 62 and inner film layer 64 can have a uniformed thickness, or may have a non-uniform thickness. For example, outer film layer 62 and inner film layer 64 can thicker at specific areas (e.g., where negative pressure manifold 40 and/or instillation manifold 42 are attached or coupled with outer film layer 62 and inner film layer 64).

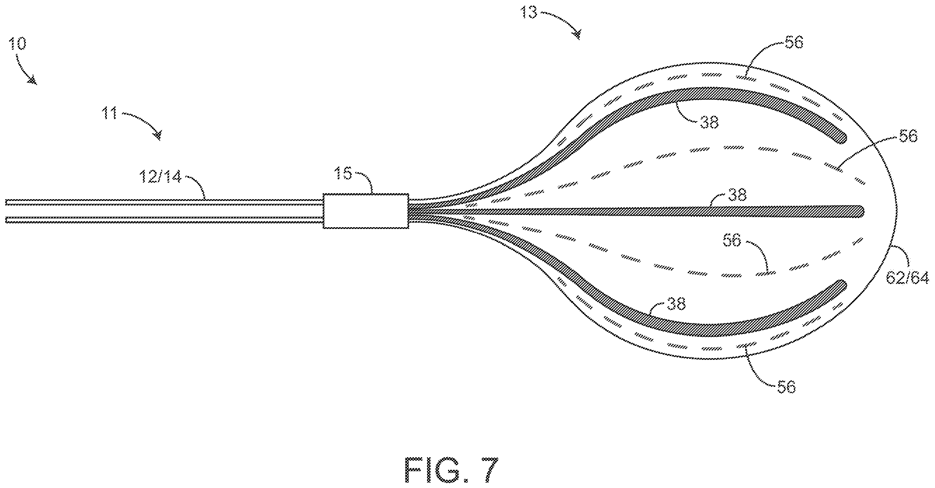

Referring particularly to FIGS. 3 and 7, dressing 13 can include one or more radio-opaque strips 56. Radio opaque strips can be printed or otherwise positioned on dressing 13. In some embodiments, radio-opaque strips 56 are positioned on one of the film layers 62 and/or 64. Radio-opaque strips 56 can be viewable with an X-ray, thereby allowing a position of dressing 13 to be viewed to ensure that dressing 13 has not shifted in the cavity. Advantageously, radio-opaque strips 56 facilitate allowing placement, alignment, and re-positioning of dressing 13. A technician or caregiver can use an X-ray to determine if dressing 13 should be repositioned or if dressing 13 has maintained a proper position within the cavity.

Referring particularly to FIGS. 5 and 6, connector 15 is shown in greater detail, according to some embodiments. Connector 15 can include a valve 66 that is selectable between a first position (shown in FIG. 5) and a second position (shown in FIG. 6). When valve 66 is in the first position, instillation fluid is provided through instillation channel 20 to instillation manifold 42. When valve 66 is in the first position, a negative pressure can be drawn through vacuum channel 22. Vacuum channel 22 may fluidly couple with negative pressure manifold 40 when in the first position. Exuded fluid from the cavity can be drawn through vacuum channel 22 as well.

When connector 15 is switched or transitioned into the second position (shown in FIG. 6), the instillation fluid enters connector 15 through instillation channel 20 but exits connector 15 through vacuum channel 22. Likewise, the exuded fluid drawn by the therapy unit or the negative pressure may enter connector 15 through instillation channel 20 from instillation manifold 42, and be delivered to the therapy unit or the negative pressure source (e.g., thereby exiting connector 15) through vacuum channel 22. In this way, connector 15 can be cross-plumbed such that a direction of fluid removal/fluid delivery can be reversed upon transitioning valve 66 between the first position and the second position. In this way, when the patient, caregiver, user, etc., reverses direction of connector 15 by selectively adjusting valve 66, a negative pressure/instillation fluid delivery direction is reversed, without requiring removal and reversal of instillation tube 12 and vacuum tube 14. Advantageously, connector 15 can be cross-plumbed to facilitate selective bidirectionality of instillation fluid delivery/fluid removal.

Referring particularly to FIG. 7, a bottom view of dressing assembly 10 is shown, according to some embodiments. As shown, dressing 13 includes three instillation pathway layers 38 that extend along a bottom side of dressing 13. Dressing 13 may also include three negative pressure pathway layers 36 that extend along dressing 13 similarly to instillation pathway layers 38 but along a top side of dressing 13. In some embodiments, instillation pathway layer 38 and negative pressure pathway layers 36 extend on opposite sides of dressing 13. In other embodiments, instillation pathway layers 38 and negative pressure pathway layers 36 are alternated such that each instillation pathway layer 38 is positioned between a pair of negative pressure pathway layers 36 and such that each negative pressure pathway layer 36 is positioned between a pair of instillation pathway layers 38.

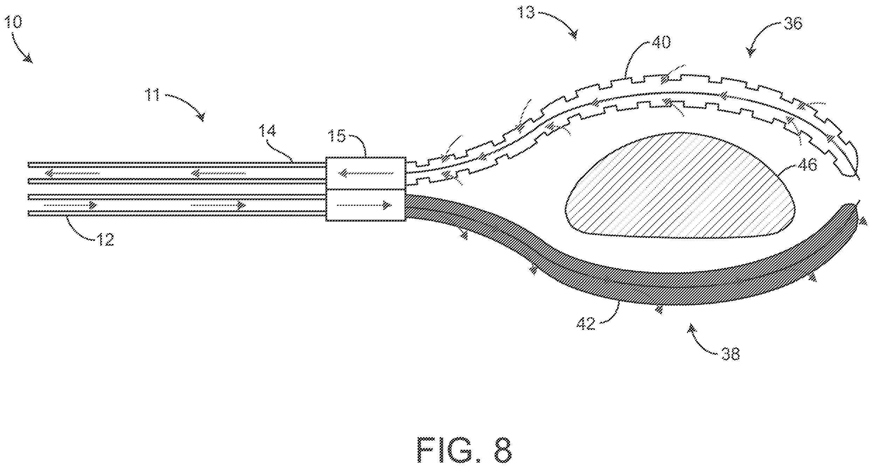

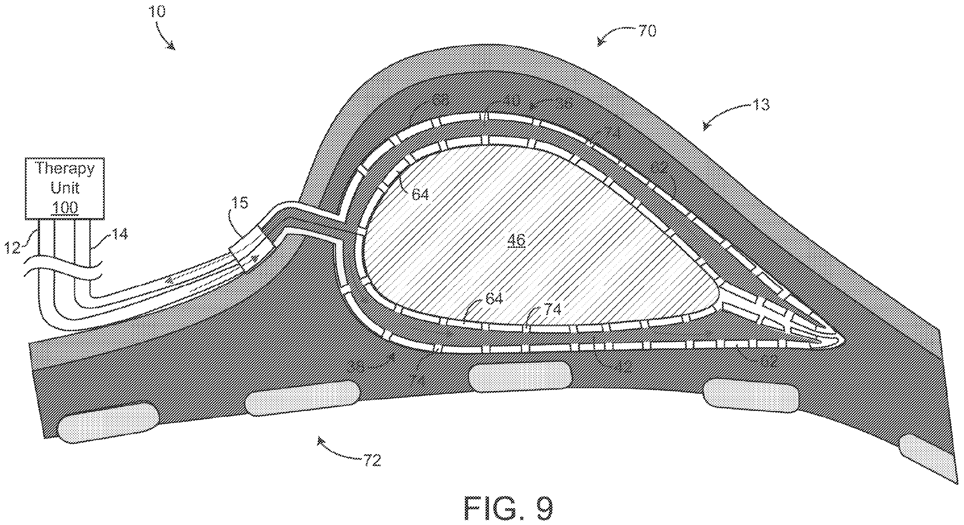

Referring particularly to FIGS. 8 and 9, fluid flow paths of dressing assembly 10 are shown, according to some embodiments. Specifically, an instillation fluid flow path and an exuded fluid flow path are shown. The instillation fluid flow path extends through instillation tube 12, connector 15, and instillation manifold 42 of instillation pathway layer 38. The instillation fluid flows through instillation tube 12, connector 15, and is provided to instillation manifold 42. The instillation fluid may saturate instillation manifold 42 and may exit instillation manifold 42 into the cavity. In some embodiments, the instillation fluid is driven to exit instillation manifold 42 into the cavity due to the negative pressure in the cavity.

Exuded fluid (e.g., instillation fluid) is drawn into negative pressure manifold 40 from within the cavity. The exuded fluid is then transferred through negative pressure manifold 40 due to the negative pressure drawn therethrough. The exuded fluid may be absorbed, wicked, or otherwise transferred through negative pressure manifold 40. The exuded fluid is then transferred through connector 15 to vacuum tube 14. The exuded fluid is transferred through vacuum tube 14 to a canister, an exuded fluid container, etc. The exuded fluid can be drawn out of the cavity and transferred to the canister due to the negative pressure drawn by the therapy unit.

Referring particularly to FIG. 9, dressing assembly 10 is shown positioned within a cavity 68 of a patient's breast 70, according to some embodiments. The instillation fluid can be transferred to cavity 68 from a therapy unit 100 through instillation tube 12. The instillation fluid is then provided to cavity 68 through instillation manifold 42. The instillation fluid may transfer through openings, holes, apertures, perforations, fenestrations, etc., shown as openings 74. Openings 74 extend through outer film layer 62 and/or inner film layer 64 of instillation pathway layer 38 and film layers 62 and 64 of negative pressure pathway layer 36 to fluidly couple instillation manifold 42 and negative pressure manifold 40 with cavity 68. In some embodiments, openings 74 extend through only outer film layer 62. Therapy unit 100 may be a PREVENA^{™} Incision Management System, a V.A.C.ULTA^{™} Therapy System, etc.

The instillation fluid is provided to cavity 68 through openings 74. The instillation fluid can then wash, flush, treat, sterilize, etc., cavity 68, tissues surrounding cavity 68, space filler 46, etc. The instillation fluid can be drawn through openings 74 of outer film layer 62 of negative pressure pathway layer 62 and removed from cavity 68 along the exuded fluid flow path through negative pressure manifold 40. The instillation fluid can be drawn along the exuded fluid flow path with exuded wound fluid. The fluid (e.g., the instillation fluid and/or the exuded fluid) is then transferred or drawn to therapy unit 100 through vacuum tube 14.

In this way, a wash direction of fluid can be produced within cavity 68. For example, the wash direction may be a general direction that fluid is provided/removed from cavity 68. The instillation fluid can be provided on a first side or along a first end of dressing 13, and drawn through cavity 68 to a second or opposite side where the fluid is removed from cavity 68. In this way, fluid within cavity 68 may be provided on the first side or at the first end, flow to the opposite side or the opposite end, and be removed from cavity 68 at the opposite side or the opposite end. The direction that the fluid generally flows in within cavity 68 is the "wash direction." Operating connector 15 to cross-plumb the vacuum tube 14 and the instillation tube 12 can result in selectively reversing the wash direction within cavity 68 such that instillation fluid is provided at the second side or the second end of cavity 68 and removed from cavity 68 at the first side or at the first end.

The first side can be a side or end of dressing 13 that faces or is directed towards ribs 72 of the patient. For example, instillation pathway layer 38 may face inwards (e.g., towards the patient's ribs 72), while negative pressure pathway layers 36 can face outwards (e.g., away from the patient's ribs 72). In this way, instillation fluid can be provided to cavity 68 at the spine-facing side of dressing 13, drawn to the front side of dressing 13, and removed from cavity 68.

### Inflatable Dressing

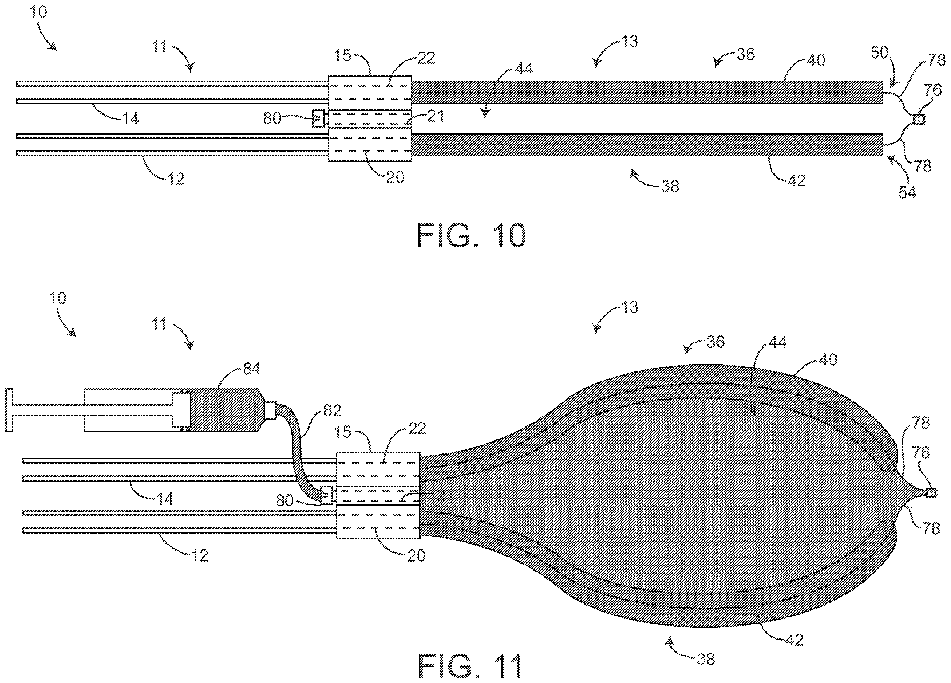

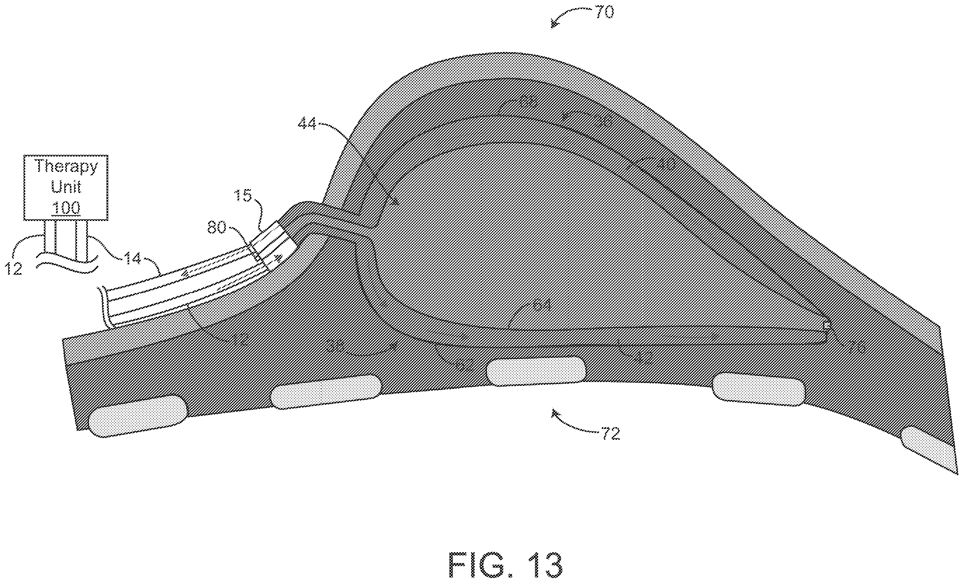

Referring particularly to FIGS. 10-13, dressing assembly 10 is shown, according to another embodiment not according to the invention. Specifically, dressing assembly 10 is shown as an inflatable dressing. Dressing assembly 10 can be the same as or similar to dressing assembly 10 as described in greater detail above with reference to FIGS. 1-9. Specifically, dressing assembly 10 includes negative pressure pathway layer 36, instillation pathway layer 38, vacuum tube 14, instillation tube 12, and connector 15.

Negative pressure pathway layer 36 and instillation pathway layer 38 can be joined, welded, sealed, etc. with each other at their distal ends 50 and 54. Negative pressure pathway layer 36 and instillation pathway layer 38 can be sealed at a weld 76. In this way, negative pressure pathway layer 36 and instillation pathway layer 38 cooperatively define a sealed inner volume 44 which can be filled with a fluid to thereby inflate dressing 13.

Negative pressure pathway layer 36 and instillation pathway layer 38 can be joined along at least a portion of their perimeter. In some embodiments, negative pressure pathway layer 36 and instillation pathway layer 38 each include an edge or outer periphery that are joined, welded, adhered, integrally formed, vacuum sealed, etc., with each other. Negative pressure pathway layer 36 and instillation pathway layer 38 can be substantially thin planar (e.g., flexible) members that cooperatively form a three-dimensional inner volume therewithin. Weld 76 may form a fluidic seal around at least a portion of the perimeters of negative pressure pathway layer 36 and instillation pathway layer 38. Negative pressure pathway layer 36 and instillation pathway layer 38 may be coupled (e.g., fluidly sealed) along different portions of their perimeters and may form a three-dimensional semi-elliptical shape.

Negative pressure pathway layer 36 and instillation pathway layer 38 can each include a film member, a layer, a polyurethane film, etc., shown as film 78. Film 78 may extend centrally through negative pressure pathway layer 36 and instillation pathway layer 38 (e.g., through instillation manifold 42 and/or negative pressure manifold 40) and may be sealed at their free ends at weld 76. In other embodiments, films 78 are outer film layers 62, while in other embodiments, films 78 are inner film layers 64. In some embodiments, weld 76 is an RF/HF weld. Instillation manifold 42 and/or negative pressure manifold 40 can be manufactured from a felted or compressed foam. The felted foam may be configured to stretch or expand as dressing 13 is inflated.

Referring particularly to FIG. 10, connector 15 is shown to include vacuum channel 22, instillation channel 20, and a fill channel 21, according to some embodiments. Vacuum channel 22 is configured to fluidly couple vacuum tube 144 with negative pressure pathway layer 36, while instillation channel 20 is configured to fluidly couple instillation tube 12 with instillation pathway layer 38. Fill channel 21 can extend through connector 15 and may fluidly couple with inner volume 44 of dressing 13. Inner volume 44 can be filled with a fluid, a gas, a liquid, etc., through fill channel 21. Connector 15 can include a valve, a connection, a fill interface, etc., shown as valve 80. Valve 80 can be configured to selectively fluidly couple fill channel 21 with inner volume 44 to facilitate the transport of fluid, liquid, gas, etc., to inner volume 44. FIG. 8 shows dressing 13 in a deflated state (e.g., without any fluid, liquid, or gas added to inner volume 44). Valve 80 can be configured to selectively fluidly couple with a corresponding tubular member, hose, conduit, etc., to selectively fluidly couple the corresponding tubular member with inner volume 44 to facilitate filling and inflating dressing 13.

Referring particularly to FIG. 11, dressing assembly 10 is shown in an inflated or filled state, according to some embodiments. In some embodiments, dressing 13 can be filled by selectively coupling (e.g., fluidly coupling) a fill apparatus 84 with valve 80. Specifically, fill apparatus 84 (e.g., a syringe, a pump, a hose, a fill system, a pumping system including a fluid reservoir, etc.) can be fluidly coupled with inner volume 44 of dressing 13 through a tubular member 82. Fill apparatus 84 is configured to deliver fluid, liquid, or gas (e.g., air, saline solution, a sterile liquid, etc.) to inner volume 44 through connector 15. The fluid may flow through tubular member 82, valve 80, fill channel 21 of connector 15, and enter inner volume 44 of dressing 13, thereby inflating dressing 13. Inner volume 44 may increase as fluid enters, thereby driving negative pressure pathway layer 36 and instillation pathway layer 38 to expand, bend, deform, etc. The fluid used to fill inner volume 44 and thereby inflate dressing 13 can be a saline solution (e.g., 0.9% saline) which is safe in the case of leakage.

In some embodiments, valve 80 is a one-way valve that facilitates unidirectional flow of fluid into inner volume 44. For example, valve 80 can be configured to allow fluid to flow in a direction such that the fluid enters inner volume 44, while preventing the fluid from flowing in an opposite direction (e.g., preventing fluid from leaking out of inner volume 44 through valve 80). Valve 80 can also be selectively coupled with connector 15 such that valve 80 can be removed, replaced, etc. In some embodiments, valve 80 can be selectively fluidly de-coupled or removed from connector 15 such that the fluid may exit inner volume 44, thereby deflating dressing 13 and emptying inner volume 44.

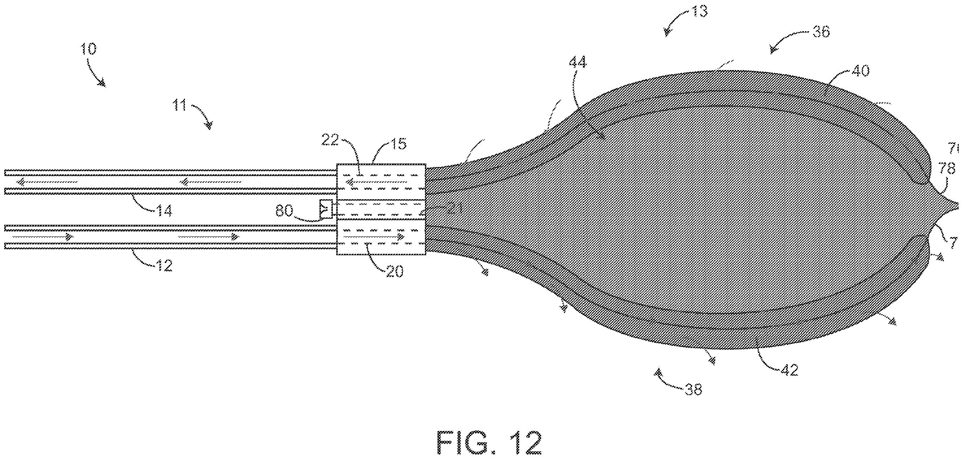

Referring particularly to FIGS. 12-13, various fluid flow paths of inflatable dressing 13 are shown, according to some embodiments. Instillation fluid may be provided to instillation tube 12 from therapy unit 100 through instillation tube 12. The instillation fluid is transferred through instillation tube 12 to connector 15. The instillation fluid is then transferred through instillation channel 20 of connector 15 to instillation pathway layer 38. The instillation fluid is transferred, absorbed, wicked, etc., through instillation manifold 42 and to cavity 68. Exuded wound fluid and/or instillation fluid is removed from cavity 68 through negative pressure pathway layer 36. The exuded wound fluid and/or the instillation fluid is transferred through negative pressure manifold 40 to connector 15. The exuded wound fluid and/or the instillation fluid is transferred through vacuum channel 22 to vacuum tube 14, where it is then transferred to therapy unit 100. The fluid may be driven to flow through negative pressure manifold 40, connector 15, and vacuum tube 14 by the negative pressure in cavity 68 produced by therapy unit 100.

In some embodiments, only the outer film layer 62 of instillation pathway layer 38 and negative pressure pathway layer 36 includes perforations, openings, holes, apertures, fenestrations, etc., to fluidly couple instillation manifold 42 and negative pressure manifold 40 with cavity 68. However, inner film layer 64 may be substantially sealed (e.g., not including perforations, holes, openings, apertures, etc.) such that the fluid within inner volume 44 is prevented or restricted from exiting inner volume 44 and entering either of instillation manifold 42 and negative pressure manifold 40. In this way, instillation fluid or fluid within cavity 68 is also prevented from entering inner volume 44 through instillation manifold 42 and/or negative pressure manifold 40. Advantageously, inner film layers 64 facilitate providing a barrier layer between negative pressure manifold 40 and instillation manifold 42 to prevent the transfer of fluid out of or into inner volume 44.

In some embodiments, dressing 13 is inflated (e.g., by filling inner volume 44 with fluid) in order to preserve a shape or volume of the patient's breast, thereby reducing patient distress. Advantageously, dressing 13 can be used to maintain symmetry between the patients breasts (e.g., after reconstructive breast surgery, breast cancer surgery, etc.), thereby maintaining patient comfort and reducing patient distress.

### Permanent Implant Negative Pressure System

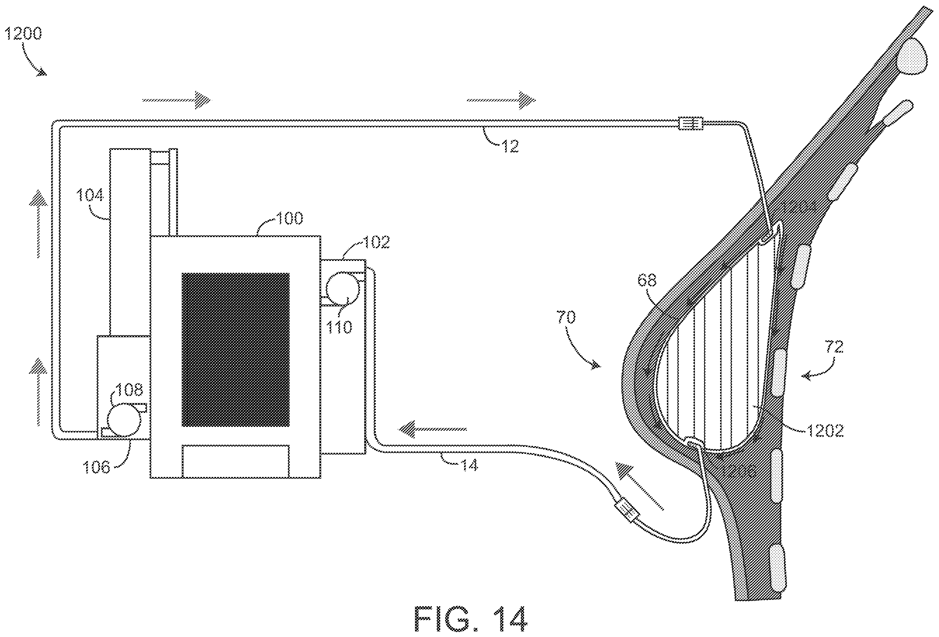

Referring particularly to FIG. 14, a negative pressure system 1200 for a permanent implant 1202 is shown, according to some embodiments not according to the invention. Negative pressure system 1200 includes therapy unit 100, instillation tube 12, and vacuum tube 14. Therapy unit 100 includes a negative pressure canister 102 that is configured to fluidly couple with vacuum tube 14 to draw a negative pressure at cavity 68 of a patient's breast 70. Permanent implant 1202 is positioned within cavity 68. Negative pressure system 1200 can be used to wash permanent implant 1202 without removing implant 1202 from cavity 68. Advantageously, this allows treatment and negative pressure wound therapy application to permanent implant 1202 without invasively removing permanent implant 1202 which may be distressing to the patient. This is intended to reduce the infection and salvage permanent implant 1202 without requiring the patient to have the entire permanent implant 1202 removed.

Vacuum tube 14 can be fluidly coupled with cavity 68 at a bottom portion of cavity 68 through a connector 1206. Instillation tube 12 can be fluidly coupled with cavity 68 at an upper portion of cavity 68 through connector 1204. Therapy unit 100 operates to provide instillation fluid into cavity 68 through instillation tube 12 and connector 1204 (e.g., at the upper portion of cavity 68). Therapy unit 100 also operates to draw fluid and a negative pressure at or from cavity 68 through vacuum tube 14 and connector 1206 at the bottom portion of cavity 68. In other embodiments, vacuum tube 14 is fluidly coupled with cavity 68 at the upper portion of cavity 68, while instillation tube 12 is fluidly coupled with cavity 68 at the bottom portion of cavity 68.

The instillation fluid can be provided at the upper portion, and flow through cavity 68 between an interior facing surface of tissue at cavity 68 and an exterior facing surface of permanent implant 1202. The instillation fluid may wash, sterilize, clean, etc., tissue at or near cavity 68 in addition to permanent implant 1202. The instillation fluid and any other fluid in cavity 68 is then removed from cavity 68 through vacuum tube 14 and connector 1206. In this way, the instillation fluid can flow from the upper portion of cavity 68 where it is provided by negative pressure system 1200 to the bottom portion of cavity 68 where is it removed. Likewise, if instillation tube 12 is fluidly coupled with cavity 68 at the bottom portion of cavity 68, and vacuum tube 14 is fluidly coupled with cavity 68 at the upper portion of cavity 68, the instillation fluid may flow upwards within cavity 68 (e.g., through a space defined between the exterior surface of the permanent implant 1202 and the inwards or interior facing surface of cavity 68).

Advantageously, negative pressure system 1200 can be used as a minimally invasive system to provide benefits associated with negative pressure wound therapy in addition to sterilizing or cleaning cavity 68, surrounding tissue, and permanent implant 1202. Negative pressure system 1200 can be used to treat infected breast cavities or infected permanent implants 1202 by providing negative pressure (e.g., through vacuum tube 14) in addition to instillation fluid. Removed fluid (e.g., instillation fluid and/or exuded fluid) can be stored in negative pressure canister 102 until negative pressure canister 102 is filled. Once negative pressure canister 102 is substantially filled, the exuded fluid and/or the instillation fluid can be disposed.

The instillation fluid can be stored in a fluid reservoir 104. Fluid reservoir 104 can be fluidly coupled with an instillation hanger 106 of therapy unit 100. Instillation hanger 106 can include an instillation pump (e.g., a discharge pump) 108 that is configured to drive the instillation fluid through instillation tube 12. In other embodiments, instillation hanger 106 does not include instillation pump 108 and the instillation fluid is drawn into cavity 68 through instillation tube 12 due to the negative pressure at cavity 68.

Referring still to FIG. 14, therapy unit 100 can include a vacuum pump, a negative pressure pump (e.g., a suction pump), shown as pneumatic pump 110. Pneumatic pump 110 can be fluidly coupled with vacuum tube 14 and is configured to draw a negative pressure at cavity 68 through vacuum tube 14. Pneumatic pump 110 can be configured to draw the negative pressure through vacuum tube 14 such that fluid (e.g., exuded wound fluid and/or instillation fluid) is drawn through vacuum tube 14 to negative pressure canister 102.

### Therapy Unit

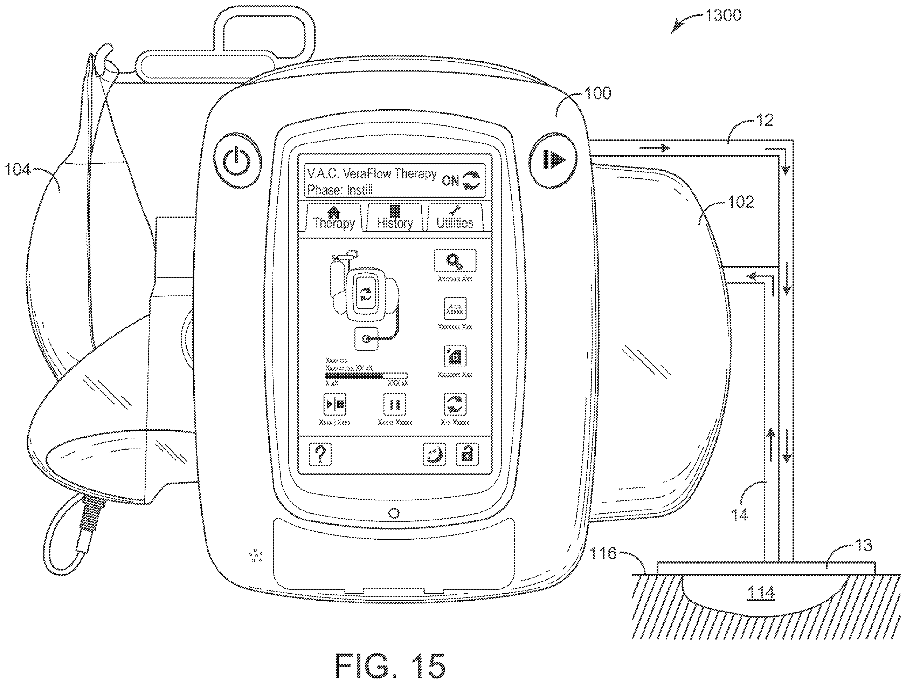

Referring now to FIGS. 15-19, a negative pressure wound therapy system 1300 is shown, according to an exemplary embodiment. System 1300 can include various components usable with any of dressing assemblies 10 as described in greater detail with reference to FIGS. 1-14. System 1300 is shown to include therapy unit 100 fluidly connected to dressing 13 via tubes 12 and 14. Dressing 13 may be adhered or sealed to a patient's skin 116 surrounding a wound 114 or may be an intra-cavity dressing as described in greater detail above with reference to FIGS. 1-14. Several examples of wound dressings 13 which can be used in combination with system 1300 are described in detail in U.S. Patent No. 7,651,484 granted January 26, 2010, U.S. Patent No. 8,394,081 granted March 12, 2013, and U.S. Patent Application No. 14/087,418 filed November 22, 2013.

Therapy unit 100 can be configured to provide negative pressure wound therapy by reducing the pressure at wound 114 or within cavity 68. Therapy unit 100 can draw a vacuum at wound 114 or within cavity 68 (relative to atmospheric pressure) by removing wound exudate, air, and other fluids from wound 114. Wound exudate may include fluid that filters from a patient's circulatory system into lesions or areas of inflammation. For example, wound exudate may include water and dissolved solutes such as blood, plasma proteins, white blood cells, platelets, and red blood cells. Other fluids removed from wound 114 and/or cavity 68 may include instillation fluid 105 previously delivered to wound 114 and/or cavity 68. Instillation fluid 105 can include, for example, a cleansing fluid, a prescribed fluid, a medicated fluid, an antibiotic fluid, or any other type of fluid which can be delivered to wound 114 or cavity 68 during wound treatment. Instillation fluid 105 may be held in an instillation fluid canister 104 and controllably dispensed to wound 114 or cavity 68 via instillation fluid tubing 12. In some embodiments, instillation fluid canister 104 is detachable from therapy unit 100 to allow canister 102 to be refilled and replaced as needed.

The fluids 107 removed from wound 114 or cavity 68 pass through vacuum tube 14 and are collected in removed fluid canister 102. Removed fluid canister 102 may be a component of therapy unit 100 configured to collect wound exudate and other fluids 107 removed from wound 114 or cavity 68. In some embodiments, removed fluid canister 102 is detachable from therapy unit 100 to allow canister 102 to be emptied and replaced as needed. A lower portion of canister 102 may be filled with wound exudate and other fluids 107 removed from wound 114 or cavity 68, whereas an upper portion of canister 102 may be filled with air. Therapy unit 100 can be configured to draw a vacuum within canister 102 by pumping air out of canister 102. The reduced pressure within canister 102 can be translated to dressing 13 and wound 114 or cavity 68 via tubing 14 such that dressing 13 and wound 114 or cavity 68 are maintained at the same pressure as canister 102.

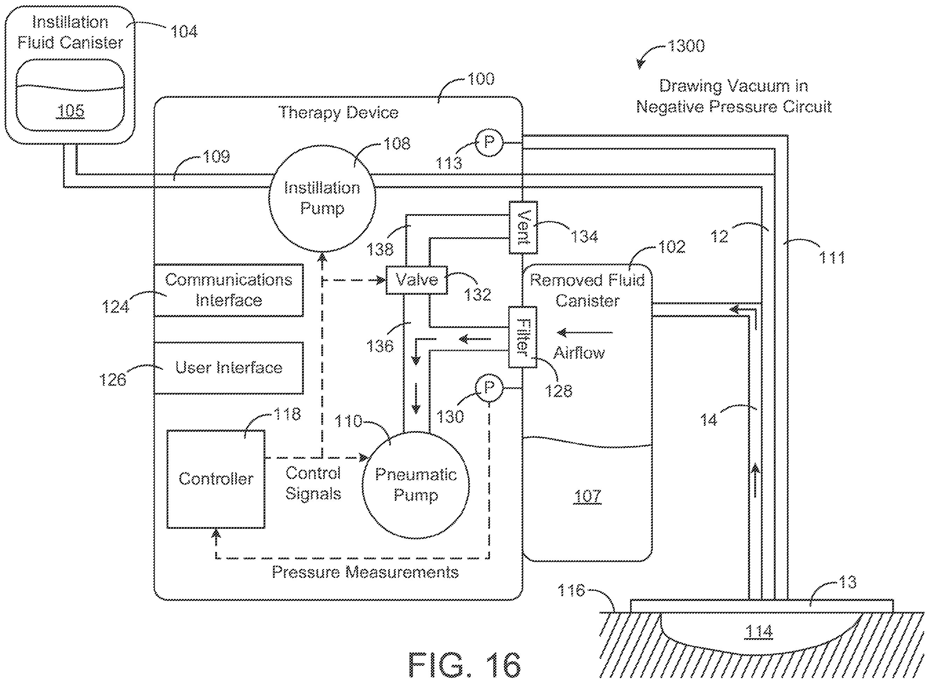

Referring particularly to FIGS. 16-17, block diagrams illustrating therapy unit 100 in greater detail are shown, according to an exemplary embodiment. Therapy unit 100 is shown to include a pneumatic pump 110, an instillation pump 108, a valve 132, a filter 128, and a controller 118. Pneumatic pump 110 can be fluidly coupled to removed fluid canister 102 (e.g., via conduit 136) and can be configured to draw a vacuum within canister 102 by pumping air out of canister 102. In some embodiments, pneumatic pump 110 is configured to operate in both a forward direction and a reverse direction. For example, pneumatic pump 110 can operate in the forward direction to pump air out of canister 102 and decrease the pressure within canister 102. Pneumatic pump 110 can operate in the reverse direction to pump air into canister 102 and increase the pressure within canister 102. Pneumatic pump 110 can be controlled by controller 118, described in greater detail below.

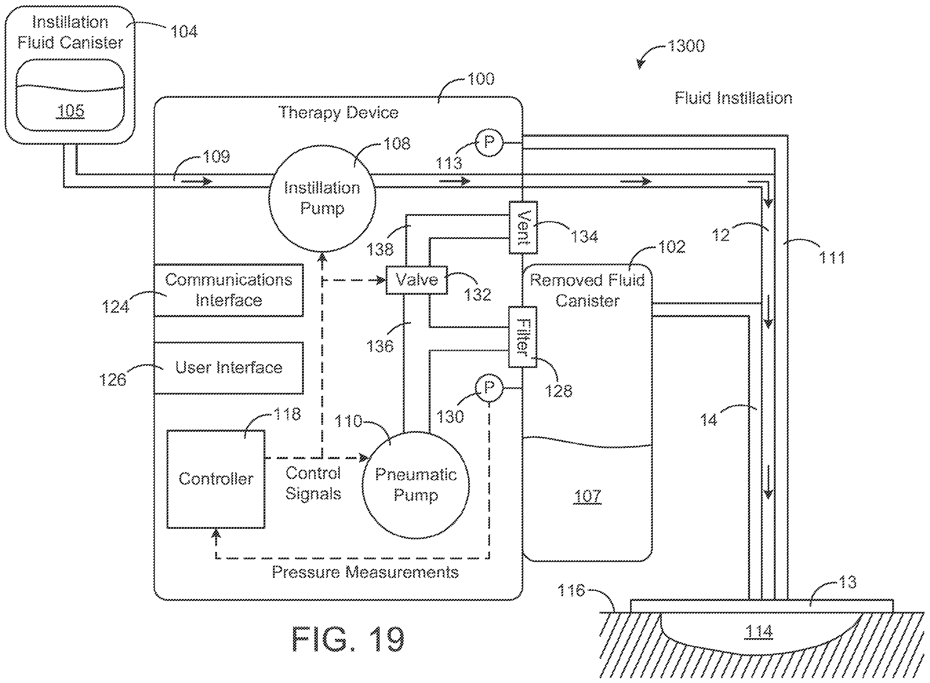

Similarly, instillation pump 108 can be fluidly coupled to instillation fluid canister 104 via tubing 109 and fluidly coupled to dressing 13 via tubing 12. Instillation pump 108 can be operated to deliver instillation fluid 105 to dressing 13 and wound 114 (or cavity 68) by pumping instillation fluid 105 through tubing 109 and tubing 12, as shown in FIG. 19. Instillation pump 108 can be controlled by controller 118, described in greater detail below.

Filter 128 can be positioned between removed fluid canister 102 and pneumatic pump 110 (e.g., along conduit 136) such that the air pumped out of canister 102 passes through filter 128. Filter 128 can be configured to prevent liquid or solid particles from entering conduit 136 and reaching pneumatic pump 110. Filter 128 may include, for example, a bacterial filter that is hydrophobic and/or lipophilic such that aqueous and/or oily liquids will bead on the surface of filter 128. Pneumatic pump 110 can be configured to provide sufficient airflow through filter 128 that the pressure drop across filter 128 is not substantial (e.g., such that the pressure drop will not substantially interfere with the application of negative pressure to wound 114 (or cavity 68) from therapy unit 100).

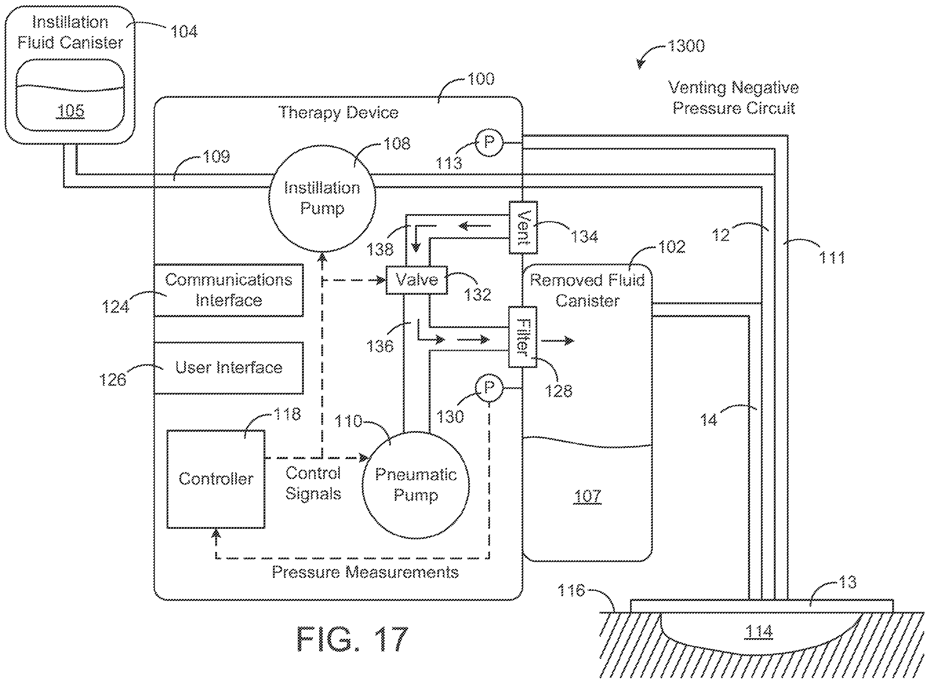

In some embodiments, therapy unit 100 operates a valve 132 to controllably vent the negative pressure circuit, as shown in FIG. 17. Valve 132 can be fluidly connected with pneumatic pump 110 and filter 128 via conduit 136. In some embodiments, valve 132 is configured to control airflow between conduit 136 and the environment around therapy unit 100. For example, valve 132 can be opened to allow airflow into conduit 136 via vent 134 and conduit 138, and closed to prevent airflow into conduit 136 via vent 134 and conduit 138. Valve 132 can be opened and closed by controller 118, described in greater detail below. When valve 132 is closed, pneumatic pump 110 can draw a vacuum within a negative pressure circuit by causing airflow through filter 128 in a first direction, as shown in FIG. 16. The negative pressure circuit may include any component of system 1300 that can be maintained at a negative pressure when performing negative pressure wound therapy (e.g., conduit 136, removed fluid canister 102, tubing 14, dressing 13, cavity 68, and/or wound 114). For example, the negative pressure circuit may include conduit 136, removed fluid canister 102, tubing 14, dressing 13, cavity 68, and/or wound 114. When valve 132 is open, airflow from the environment around therapy unit 100 may enter conduit 136 via vent 134 and conduit 138 and fill the vacuum within the negative pressure circuit. The airflow from conduit 136 into canister 102 and other volumes within the negative pressure circuit may pass through filter 128 in a second direction, opposite the first direction, as shown in FIG. 17.

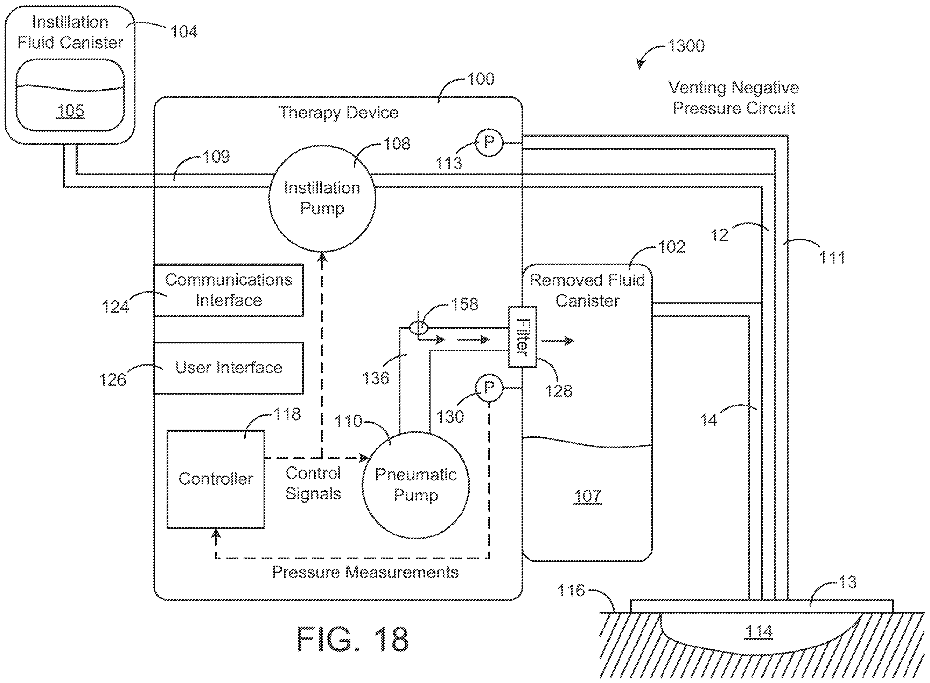

In some embodiments, therapy unit 100 vents the negative pressure circuit via an orifice 158, as shown in FIG. 18. Orifice 158 may be a small opening in conduit 136 or any other component of the negative pressure circuit (e.g., removed fluid canister 102, tubing 14, tubing 111, dressing 13, etc.) and may allow air to leak into the negative pressure circuit at a known rate. In some embodiments, therapy unit 100 vents the negative pressure circuit via orifice 158 rather than operating valve 132. Valve 132 can be omitted from therapy unit 100 for any embodiment in which orifice 158 is included. The rate at which air leaks into the negative pressure circuit via orifice 158 may be substantially constant or may vary as a function of the negative pressure, depending on the geometry of orifice 158.

In some embodiments, therapy unit 100 includes a variety of sensors. For example, therapy unit 100 is shown to include a pressure sensor 130 configured to measure the pressure within canister 102 and/or the pressure at dressing 13 or cavity 68 or wound 114. In some embodiments, therapy unit 100 includes a pressure sensor 113 configured to measure the pressure within tubing 111. Tubing 111 may be connected to dressing 13 and may be dedicated to measuring the pressure at dressing 13 or wound 114 or cavity 68 without having a secondary function such as channeling installation fluid 105 or wound exudate. In various embodiments, tubing 12, 110, and 111 may be physically separate tubes or separate lumens within a single tube that connects therapy unit 100 to dressing 13. Accordingly, tubing 14 may be described as a negative pressure lumen that functions apply negative pressure dressing 13 or wound 114 or cavity 68, whereas tubing 111 may be described as a sensing lumen configured to sense the pressure at dressing 13 or wound 114 or cavity 68. Pressure sensors 130 and 113 can be located within therapy unit 100, positioned at any location along tubing 12, 110, and 111, or located at dressing 13 in various embodiments. Pressure measurements recorded by pressure sensors 130 and/or 113 can be communicated to controller 118. Controller 118 use the pressure measurements as inputs to various pressure testing operations and control operations performed by controller 118.

Controller 118 can be configured to operate pneumatic pump 110, instillation pump 108, valve 132, and/or other controllable components of therapy unit 100. For example, controller 118 may instruct valve 132 to close and operate pneumatic pump 110 to establish negative pressure within the negative pressure circuit. Once the negative pressure has been established, controller 118 may deactivate pneumatic pump 110. Controller 118 may cause valve 132 to open for a predetermined amount of time and then close after the predetermined amount of time has elapsed.

In some embodiments, therapy unit 100 includes a user interface 126. User interface 126 may include one or more buttons, dials, sliders, keys, or other input devices configured to receive input from a user. User interface 126 may also include one or more display devices (e.g., LEDs, LCD displays, etc.), speakers, tactile feedback devices, or other output devices configured to provide information to a user. In some embodiments, the pressure measurements recorded by pressure sensors 130 and/or 113 are presented to a user via user interface 126. User interface 126 can also display alerts generated by controller 118. For example, controller 118 can generate a "no canister" alert if canister 102 is not detected.

In some embodiments, therapy unit 100 includes a data communications interface 124 (e.g., a USB port, a wireless transceiver, etc.) configured to receive and transmit data. Communications interface 124 may include wired or wireless communications interfaces (e.g., jacks, antennas, transmitters, receivers, transceivers, wire terminals, etc.) for conducting data communications external systems or devices. In various embodiments, the communications may be direct (e.g., local wired or wireless communications) or via a communications network (e.g., a WAN, the Internet, a cellular network, etc.). For example, communications interface 124 can include a USB port or an Ethernet card and port for sending and receiving data via an Ethernet-based communications link or network. In another example, communications interface 124 can include a Wi-Fi transceiver for communicating via a wireless communications network or cellular or mobile phone communications transceivers.

### Clamshell Process

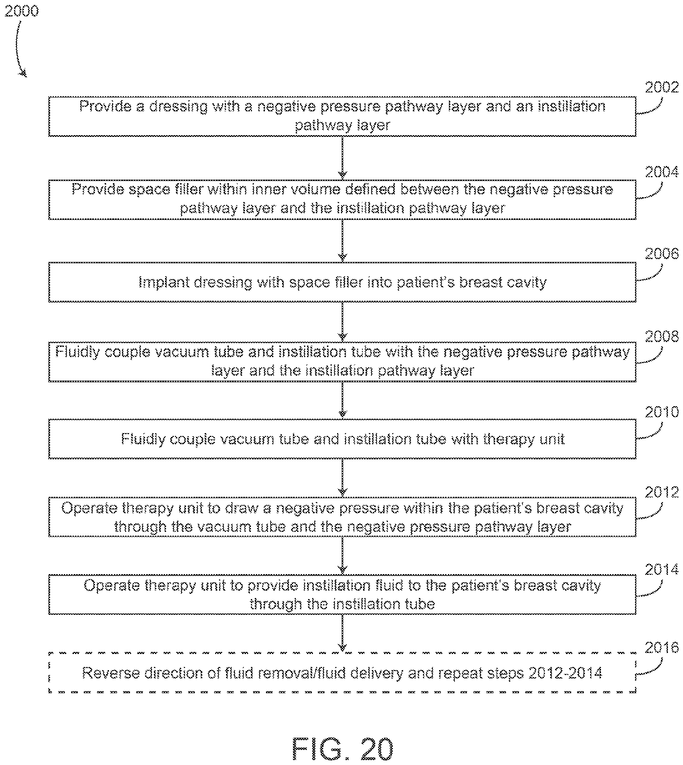

Referring now to FIG. 20, a process 2000 for using dressing assembly 10 as described in greater detail above with reference to FIGS. 1-9, according to some embodiments not according to the invention. Process 2000 includes steps 2002-2016 and can be used to provide negative pressure wound therapy and instillation fluid to a breast cavity. Process 2000 can be performed by a clinician, a caregiver, etc.

Process 2000 includes providing a dressing with a negative pressure pathway layer and an instillation pathway layer (step 2002), according to some embodiments. The negative pressure pathway layer may include a negative pressure manifold that is positioned between inner and outer film layers. The instillation pathway layer can include an instillation manifold that is positioned between inner and outer film layers. The inner and outer film layers of both the negative pressure pathway layer and the instillation pathway layer can be sealed with each other. The inner and/or the outer film layers may include perforations, openings, holes, apertures, fenestrations, etc., so that fluid passing through the manifolds can be transferred into or out of the breast cavity. Step 2002 can be performed by a caregiver or a technician. The instillation pathway layer and the negative pressure pathway layer may each be fixedly coupled at a proximate end with a connector, and may have a free distal end.

Process 2000 includes providing a space filler within an inner volume defined between the negative pressure pathway layer and the instillation pathway layer (step 2004), according to some embodiments. In some embodiments, the space filler is a breast expander, or any other medical object, implant, gauze, felt, etc., that can be positioned within the inner volume to facilitate filling the patient's breast cavity. The negative pressure pathway layer and the instillation pathway layer can be wrapped around the space filler (e.g., by a technician or a caregiver or a surgeon). For example, the free distal ends may be wrapped around the space filler.

Process 2000 includes implanting the dressing with the space filler into the patient's breast cavity (step 2006), according to some embodiments. Step 2006 can be performed by a surgeon, a caregiver, a medical professional, etc. The dressing can be implanted into the patient's breast cavity with the space filler positioned between the negative pressure pathway layer and the instillation pathway layer. Exterior or outer surfaces of the outer film layers of the negative pressure pathway layer and the instillation pathway layer can directly contact or engage an interior facing surface of the patient's breast cavity (e.g., inwards facing tissue).

Process 2000 includes fluidly coupling a vacuum tube and an instillation tube with the negative pressure pathway layer and the instillation pathway layer (step 2008), according to some embodiments. The vacuum tube and the instillation tube can be fluidly coupled with the negative pressure pathway layer and the instillation pathway layer through a connector that fluidly couples with the manifolds (e.g., the negative pressure manifold and the instillation manifold) through different channels. In this way, instillation fluid can be independently provided to the instillation manifold of the instillation pathway layer and wound fluid can be independently removed through the negative pressure manifold of the negative pressure pathway layer. Step 2008 can be performed by a caregiver. The caregiver may fluidly couple the vacuum tube and the instillation tube with the connector so that negative pressure wound therapy can be performed.

Process 2000 includes fluidly coupling the vacuum tube and the instillation tube with a therapy unit (step 2010), according to some embodiments. Step 2010 may be performed by a caregiver, a technician, a surgeon, etc. Step 2010 can be performed by fluidly coupling or removably coupling the vacuum tube and the instillation tube with corresponding connection portions of the therapy unit.

Process 2000 includes operating the therapy unit to draw a negative pressure within the patient's breast cavity through the vacuum tube and the negative pressure pathway layer (step 2012), according to some embodiments. Step 2012 can be performed by the therapy unit. The therapy unit may include a controller, a power source, and a pneumatic pump configured to operatively draw a negative pressure. The pneumatic pump can be fluidly coupled or configured to draw the negative pressure within the cavity through the vacuum tube. For example, the pneumatic pump can be fluidly coupled with the vacuum tube such that negative pressure drawn at the vacuum tube is transferred to the patient's breast cavity.

Process 2000 includes operating the therapy unit to provide instillation fluid to the patient's breast cavity through the instillation tube (step 2014), according to some embodiments. In some embodiments, step 2014 is performed concurrently with step 2012. For example, the instillation fluid may be provided to the patient's breast cavity while the negative pressure is drawn at the patient's breast cavity. The therapy unit may include an instillation pump (e.g., a discharge pump) and a fluid reservoir that contains the instillation fluid. The instillation pump can be operated by the controller to provide the instillation fluid from the fluid reservoir to the instillation tube. The instillation fluid is driven through the instillation tube, through the connector, and to the instillation manifold that is positioned between the film layers. The instillation fluid can be absorbed or transferred through the instillation manifold and may exit through the holes in either of the film layers to the patient's breast cavity. In some embodiments, the flow of the instillation fluid into the patient's breast cavity is facilitated by the negative pressure produced within the breast cavity by the pneumatic pump.

Process 2000 can also include reversing a direction of fluid removal/fluid delivery and repeating steps 2012-2014 (step 2016), according to some embodiments. Step 2016 can be performed by adjusting or operating the connector to cross-plumb the instillation tube and the vacuum tube. In this way, the instillation manifold can be used to draw the negative pressure within the cavity (e.g., by performing step 2012 with the direction reversed) and the negative pressure manifold can be used to provide instillation fluid to the cavity. The direction that fluid is added/removed to/from the cavity can be reversed by operating or cross-plumbing the connector.

### Inflatable Dressing Process

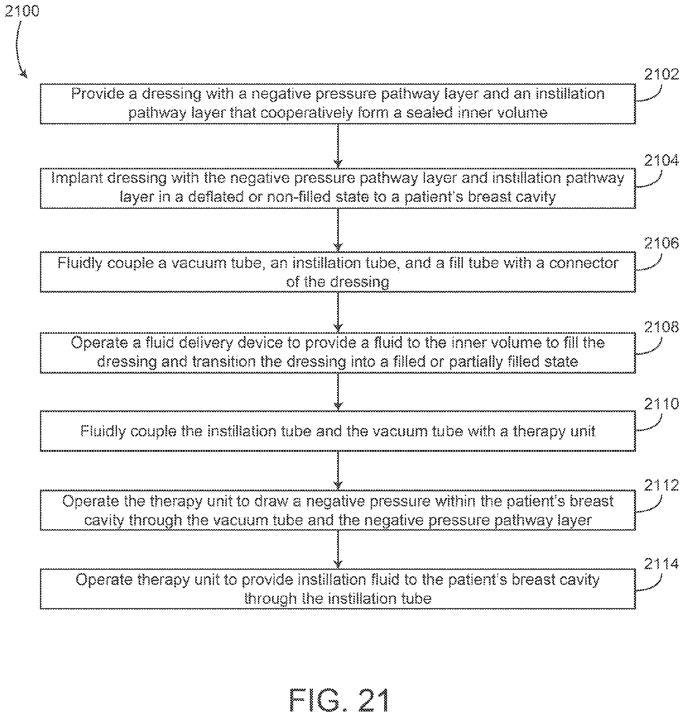

Referring now to FIG. 21, a process 2100 for using the inflatable dressing 13 as described in greater detail above with reference to FIGS. 10-13 is shown, according to some embodiments not according to the invention. Process 2100 includes steps 2102-2114, according to some embodiments. Process 2100 can be performed by a technician, a surgeon, a caregiver, etc.

Process 2100 includes providing a dressing including a negative pressure pathway layer, and an instillation pathway layer that cooperatively form or define a sealed inner volume (step 2102), according to some embodiments. In some embodiments, the dressing is the same as or similar to the dressing 13 or the dressing assembly 10 as described in greater detail above with reference to FIGS. 10-13. The negative pressure pathway layer and the instillation pathway layer can each include a manifold (e.g., a negative pressure manifold and an instillation manifold) that are positioned between an inner film layer and an outer film layer. In some embodiments, the outer film layer includes various openings, holes, apertures, perforations, fenestrations, etc., that fluidly couple the manifolds with a breast cavity in which the dressing can be implanted. The inner film layers of the negative pressure pathway layer and the instillation pathway layer may be continuous (e.g., not including any perforations or holes) to facilitate ensuring that fluid in the inner volume does not leak out.

Process 2100 includes implanting the dressing with the negative pressure pathway layer and the instillation pathway layer in a deflated or non-filled state to a patient's breast cavity (step 2104), according to some embodiments. In some embodiments, the dressing is implanted into the patient's breast cavity without any fluid present in the inner volume defined by the negative pressure pathway layer and the instillation pathway layer. In this state, the dressing may have a smaller size than when the dressing is filled with fluid. The dressing may have the form of a compressible or inflatable bladder, balloon, etc. Step 2104 can be performed by a technician, a caregiver, a surgeon, etc., or any other medical professional.

Process 2100 includes fluidly coupling a vacuum tube, an instillation tube, and a fill tube with a connector of the dressing (step 2106), according to some embodiments. The vacuum tube, the instillation tube, and the fill tube can be fluidly coupled with different channels of the connector. The vacuum tube can be fluidly coupled with the negative pressure manifold that is positioned between the film layers of the negative pressure pathway layer. The instillation tube can be fluidly coupled with the instillation manifold that is positioned between the film layers of the instillation pathway layer. The fill tube can be fluidly coupled with a valve at the connector (e.g., one-way valve) so that the fill tube is fluidly coupled with the inner volume of the dressing defined between the instillation pathway layer and the negative pressure pathway layer. Step 2106 can be performed by a technician, a caregiver, etc., after the dressing has been implanted in the patient's breast cavity.

Process 2100 includes operating a fluid delivery device to provide a fluid to the inner volume through the fill tube and the connector to fill the dressing and transition the dressing into a filled or partially filled state (step 2108), according to some embodiments. Step 2108 can be performed by a caregiver, a technician, a surgeon, etc. The fluid delivery device can be a syringe, a pump, etc., or any other device configured to pressure a fluid (e.g., a liquid or a gas) to drive the fluid into the inner volume of the dressing. The technician or the caregiver or the surgeon can operate the fluid delivery device until the dressing is filled to a desired state.

Process 2100 includes fluidly coupling the instillation tube and the vacuum tube with a therapy unit (step 2110), according to some embodiments. In some embodiments, step 2110 is the same as or similar to step 2010 of process 2000 as described in greater detail above with reference to FIG. 20.

Process 2100 includes operating the therapy unit to draw a negative pressure within the patient's breast cavity through the vacuum tube and the negative pressure pathway layer (step 2112) and operating the therapy unit to provide instillation fluid to the patient's breast cavity through the instillation tube (step 2114), according to some embodiments. Steps 2112 and 2114 can be the same as or similar to steps 2012 and 2014 of process 2000 as described in greater detail above with reference to FIG. 20.

### Permanent Implant Treatment Process

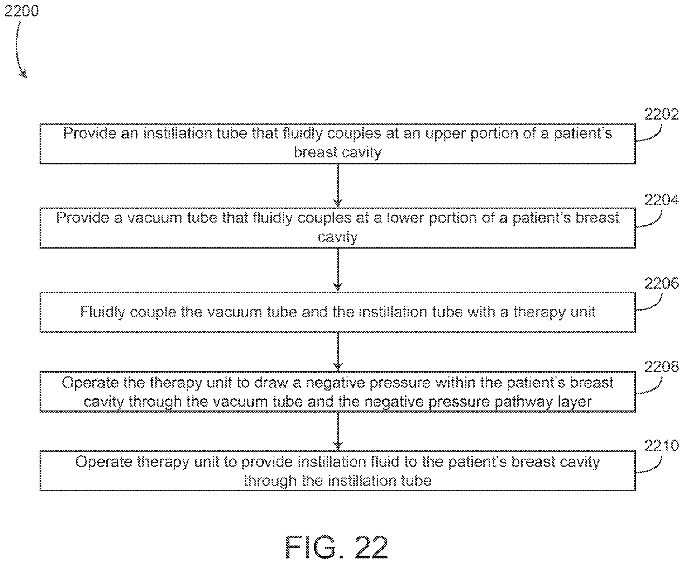

Referring now to FIG. 22, a process 2200 for treating a permanent implant is shown, according to some embodiments not according to the invention. Process 2200 includes steps 2202-2114, according to some embodiments. Process 2200 can be performed by a technician, a caregiver, a surgeon, etc. In some embodiments, process 2200 is performed with negative pressure system 1200.

Process 2200 includes providing an instillation tube that fluidly couples at an upper portion of a patient's breast cavity (step 2202), according to some embodiments. The instillation tube can be configured to deliver instillation fluid (e.g., a saline solution, a sterile solution, etc.) to the patient's breast cavity. The instillation tube can be provided and fluidly coupled with the upper portion of the patient's breast cavity by a surgeon, a medical professional, etc. The instillation tube may be surgically inserted so that an open end of the instillation tube fluidly couples with the upper portion of the patient's breast cavity. A permanent implant may be positioned within the patient's breast cavity, and the instillation tube can be configured to deliver or provide instillation fluid to the patient's breast cavity while the permanent implant is present. The instillation tube can be surgically inserted or fluidly coupled with the patient's breast cavity while the permanent implant is present and without removing (e.g., surgically) the permanent implant.

Process 2200 includes providing a vacuum tube that fluidly couples at a lower portion of a patient's breast cavity (step 2204), according to some embodiments. The vacuum tube can be inserted, surgically implanted, etc., at the lower portion of the patient's breast cavity. The vacuum tube can be configured to draw a negative pressure within the patient's breast cavity in addition to drawing out exuded fluid (e.g., wound fluid) or instillation fluid from the patient's breast cavity. The vacuum tube can be provided and fluidly coupled with the lower portion of the patient's breast cavity by a surgeon, a medical professional, etc. The vacuum tube may be surgically inserted so that an open end of the vacuum tube fluidly couples with the lower portion of the patient's breast cavity. The vacuum tube can be inserted while the permanent implant is still present within the patient's breast cavity, without requiring removal of the permanent implant. The vacuum tube can be configured to draw fluid from between an interior surface of the patient's breast cavity and an exterior surface of the permanent implant.

Process 2200 includes fluidly coupling the vacuum tube and the instillation tube with a therapy unit (step 2206), according to some embodiments. The therapy unit can include a pneumatic pump configured to fluidly couple with the vacuum tube and draw a negative pressure at the patient's breast cavity. The therapy unit may also include an instillation pump that is configured to drive or deliver instillation fluid from the therapy unit to the patient's breast cavity through the instillation tube. Step 2206 can be performed by a medical professional, a caregiver, a surgeon, etc. The instillation tube and the vacuum tube can be removably fluidly coupled with the therapy unit.

Process 2200 includes operating the therapy unit to draw a negative pressure within the patient's breast cavity through the vacuum tube and the negative pressure pathway layer (step 2208), according to some embodiments. The negative pressure may be drawn at the patient's breast cavity by operating the pneumatic pump that is fluidly coupled with the vacuum tube. In some embodiments, the therapy unit includes a controller that is configured to operate the pneumatic pump to draw the negative pressure. The negative pressure at the patient's breast cavity may originate at the bottom portion of the patient's breast cavity where the vacuum tube is fluidly coupled.

Process 2200 includes operating the therapy unit to provide instillation fluid to the patient's breast cavity through the instillation tube (step 2210), according to some embodiments. The instillation pump can be operated to drive instillation fluid through the instillation tube to the patient's breast cavity. The instillation fluid is provided into the patient's breast cavity at the upper portion of the patient's breast cavity. Steps 2208 can be performed concurrently with each other. The instillation fluid may also be driven to enter the patient's breast cavity from the therapy unit due to the negative pressure drawn within the patient's breast cavity by the pneumatic pump. The instillation fluid may be provided to the patient's breast cavity at the upper portion of the patient's breast cavity, flow along a space defined between the exterior surface of the permanent implant and the interior surface of the breast cavity, and be removed through the vacuum tube at the bottom portion of the patient's breast cavity.

### Temporary Breast Implant

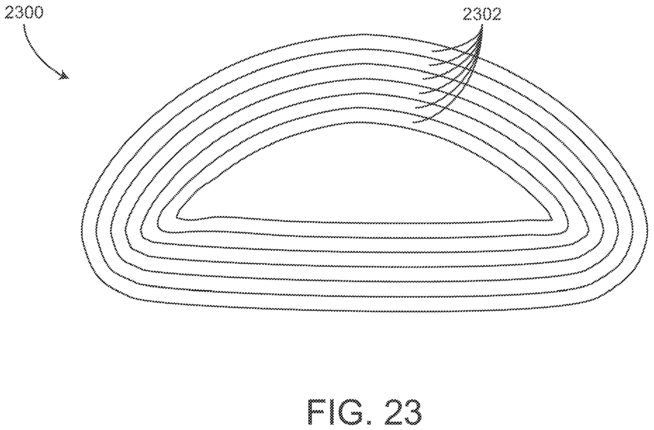

Referring particularly to FIG. 23, a temporary breast implant 2300 is usable with any of the dressings, systems, etc., described in greater detail above with reference to FIGS. 1-22. Temporary breast implant 2300 can be implanted into a patient's breast cavity for temporary and/or permanent applications. Temporary breast implant 2300 can include various layers 2302 of filler material. Layers 2302 may be removably coupled with adjacent layers so that an outermost layer can be peeled off and so that temporary breast implant 2300 can be shaped, formed, re-shaped, re-sized, etc., as desired. Layers 2302 can be a foam material (e.g., V.A.C.^{®} GRANUFOAM^{™}) that are molded to each other to create a foam implant shaped wound filler. In the case of an infected breast implant, temporary breast implant 2300 can be shaped similar to that of a typical breast expander (used to stretch breast skin post mastectomy). Layers 2302 can be peeled away to resize temporary breast implant 2300 until temporary breast implant 2300 is a desired or required size (e.g., so that it is the same size as an implant being removed). In some embodiments, temporary breast implant 2300 may shrink under negative pressure. Temporary breast implant 2300 can be sized so that it is slightly larger than required, thereby accounting for shrinkage that occurs when negative pressure is applied and thereby ensuring that the patient's breasts will be symmetrical during therapy.

### Configuration of Exemplary Embodiments

As utilized herein, the terms "approximately," "about," "substantially," and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the invention as recited in the appended claims.

It should be noted that the terms "exemplary" and "example" as used herein to describe various embodiments is intended to indicate that such embodiments are possible examples, representations, and/or illustrations of possible embodiments (and such term is not intended to connote that such embodiments are necessarily extraordinary or superlative examples).

The terms "coupled," "connected," and the like, as used herein, mean the joining of two members directly or indirectly to one another. Such joining may be stationary (e.g., permanent, etc.) or moveable (e.g., removable, releasable, etc.). Such joining may be achieved with the two members or the two members and any additional intermediate members being integrally formed as a single unitary body with one another or with the two members or the two members and any additional intermediate members being attached to one another.

References herein to the positions of elements (e.g., "first", "second", "primary," "secondary," "above," "below," "between," etc.) are merely used to describe the orientation of various elements in the figures. It should be noted that the orientation of various elements may differ according to other exemplary embodiments, and that such variations are intended to be encompassed by the present disclosure.

The construction and arrangement of the systems and methods as shown in the various exemplary embodiments are illustrative only. Although only a few embodiments have been described in detail in this disclosure, many modifications are possible (e.g., variations in sizes, dimensions, structures, shapes and proportions of the various elements, values of parameters, mounting arrangements, use of materials, colors, orientations, etc.). For example, the position of elements may be reversed or otherwise varied and the nature or number of discrete elements or positions may be altered or varied. Accordingly, all such modifications are intended to be included within the scope of the present disclosure. The order or sequence of any process or method steps may be varied or resequenced according to alternative embodiments. Other substitutions, modifications, changes, and omissions may be made in the design, operating conditions and arrangement of the exemplary embodiments without departing from the scope of the present disclosure.

## Claims

1. A dressing for an internal cavity, the dressing comprising:
a connector;
a negative pressure pathway layer coupled with the connector at a first portion of the negative pressure pathway layer, wherein a portion of the negative pressure pathway layer is substantially free;
an instillation pathway layer coupled with the connector at a first portion of the instillation pathway layer, wherein a portion of the instillation pathway layer is substantially free;
a negative pressure manifold disposed within the negative pressure pathway layer, wherein a first portion of the negative pressure manifold is fluidly coupled with a first channel of the connector; and
an instillation manifold disposed within the instillation pathway layer, wherein a portion of the instillation manifold is fluidly coupled with a second channel of the connector;
wherein the negative pressure pathway layer and the instillation pathway layer are sealed along one or more portions of a perimeter of the negative pressure pathway layer and a perimeter of the instillation pathway layer and are configured to cooperatively form an inner volume therebetween, wherein the inner volume is configured to receive a space filler; and
wherein the negative pressure pathway layer, the instillation pathway layer, and the space filler are collectively configured to be positioned within the internal cavity.

2. The dressing of Claim 1, wherein the connector is configured to fluidly couple with a first tubular member at the first channel and a second tubular member at the second channel, wherein the first channel is fluidly coupled with the first portion of the negative pressure pathway layer and the second channel is fluidly coupled with the first portion of the instillation pathway layer, wherein the first tubular member, the first channel, and the negative pressure pathway layer are configured for negative pressure application to the internal cavity and fluid removal from the internal cavity.

3. The dressing of Claim 2, wherein the second tubular member, the second channel, and the instillation pathway layer are configured for fluid instillation to the internal cavity.

4. The dressing of Claim 1, wherein the first channel and the second channel extend through the connector.

5. The dressing of Claim 1, wherein the internal cavity is an internal cavity of a patient's breast.

6. The dressing of Claim 1, wherein the negative pressure pathway layer and the instillation pathway layer are opposite sides of the dressing.

7. The dressing of Claim 1, wherein the negative pressure pathway layer comprises a top film layer and a bottom film layer with the negative pressure manifold disposed therebetween, wherein the top film layer and bottom film layer are polyurethane films and an outer one of the top film layer and the bottom film layer of the negative pressure pathway layer is perforated or fenestrated.

8. The dressing of Claim 1, wherein the instillation pathway layer comprises a top film layer and a bottom film layer with the instillation manifold disposed therebetween, wherein the top film layer and the bottom film layer of the instillation pathway layer are polyurethane films and an outer one of the top film layer and the bottom film layer of the instillation pathway layer is perforated or fenestrated.

9. The dressing of Claim 1, wherein the space filler is a temporary or permanent breast implant or an inflatable bladder or balloon.

10. The dressing of Claim 1, wherein the negative pressure manifold and the instillation manifold comprise foam webbings.

11. The dressing of Claim 1, wherein the negative pressure manifold is any of a textile, a webbed material, a gauze, a vacuum formed structure, or an embossed structure.

12. The dressing of Claim 1, wherein the instillation manifold has a hydrophilicity that is greater than a hydrophilicity of the negative pressure manifold.

13. The dressing of Claim 1, further comprising one or more radio-opaque strips or printed ink strips on the dressing.

14. The dressing of Claim 1, wherein the connector comprises a valve configured to selectively cross-connect the first channel with the instillation pathway layer and the second channel with the negative pressure pathway layer to reverse a direction of fluid flow in the internal cavity.

15. The dressing of Claim 1, wherein the connector is configured to fluidly couple with a therapy unit, wherein the therapy unit comprises:
a negative pressure pump configured to fluidly couple with the first channel to draw a negative pressure at the internal cavity; and
an instillation pump configured to fluidly couple with the second channel to provide instillation fluid to the internal cavity.

## Patentansprüche

1. Ein Verband für eine innere Kavität, der Verband aufweisend:
ein Verbinder;
eine Unterdruckpfadschicht, die an einem ersten Abschnitt der Unterdruckpfadschicht mit dem Verbinder gekoppelt ist, wobei ein Abschnitt der Unterdruckpfadschicht im Wesentlichen frei ist;
eine Instillationspfadschicht, die an einem ersten Abschnitt der Instillationspfadschicht mit dem Verbinder gekoppelt ist, wobei ein Abschnitt der Instillationspfadschicht im Wesentlichen frei ist;
einen Unterdruckverteiler, der innerhalb der Unterdruckpfadschicht angeordnet ist, wobei ein erster Abschnitt des Unterdruckverteilers mit einem ersten Kanal des Verbinders fluidisch gekoppelt ist; und
einen Instillationsverteiler, der innerhalb der Instillationspfadschicht angeordnet ist, wobei ein Abschnitt des Instillationsverteilers mit einem zweiten Kanal des Verbinders fluidisch gekoppelt ist;
wobei die Unterdruckpfadschicht und die Instillationspfadschicht entlang einem oder mehreren Abschnitten eines Umfangs der Unterdruckpfadschicht und eines Umfangs der Instillationspfadschicht abgedichtet sind und konfiguriert sind, um zusammen ein Innenvolumen dazwischen auszubilden, wobei das Innenvolumen konfiguriert ist, um einen Raumfüller aufzunehmen; und
wobei die Unterdruckpfadschicht, die Instillationspfadschicht und der Raumfüller gemeinsam konfiguriert sind, um innerhalb der inneren Kavität positioniert zu werden.

2. Der Verband nach Anspruch 1, wobei der Verbinder konfiguriert ist, um mit einem ersten röhrenförmigen Element an dem ersten Kanal und einem zweiten röhrenförmigen Element an dem zweiten Kanal fluidisch zu koppeln, wobei der erste Kanal mit dem ersten Abschnitt der Unterdruckpfadschicht fluidisch gekoppelt ist und der zweite Kanal mit dem ersten Abschnitt der Instillationspfadschicht fluidisch gekoppelt ist, wobei das erste röhrenförmige Element, der erste Kanal und die Unterdruckpfadschicht für eine Anwendung von Unterdruck auf die innere Kavität und eine Entfernung von Fluid aus der inneren Kavität konfiguriert sind.

3. Der Verband nach Anspruch 2, wobei das zweite röhrenförmige Element, der zweite Kanal und die Instillationspfadschicht für eine Fluidinstillation in der inneren Kavität konfiguriert sind.

4. Der Verband nach Anspruch 1, wobei der erste Kanal und der zweite Kanal durch den Verbinder hindurch verlaufen.

5. Der Verband nach Anspruch 1, wobei die innere Kavität eine innere Kavität der Brust eines Patienten ist.

6. Der Verband nach Anspruch 1, wobei sich die Unterdruckpfadschicht und die Instillationspfadschicht auf gegenüberliegenden Seiten des Verbandes befinden.

7. Der Verband nach Anspruch 1, wobei die Unterdruckpfadschicht eine obere Folienschicht und eine untere Folienschicht aufweist, wobei der Unterdruckverteiler dazwischen angeordnet ist, wobei die obere Folienschicht und die untere Folienschicht Polyurethanfolien sind und eine äußere der oberen Folienschicht und der unteren Folienschicht der Unterdruckpfadschicht perforiert oder fenestriert ist.

8. Der Verband nach Anspruch 1, wobei die Instillationspfadschicht eine obere Folienschicht und eine untere Folienschicht aufweist, wobei der Instillationsverteiler dazwischen angeordnet ist, wobei die obere Folienschicht und die untere Folienschicht der Instillationspfadschicht Polyurethanfolien sind und eine äußere der oberen Folienschicht und der unteren Folienschicht der Instillationspfadschicht perforiert oder fenestriert ist.

9. Der Verband nach Anspruch 1, wobei der Raumfüller ein temporäres oder permanentes Brustimplantat oder eine aufblasbare Blase oder ein aufblasbarer Ballon ist.

10. Der Verband nach Anspruch 1, wobei der Unterdruckverteiler und der Instillationsverteiler Schaumstoffgewebe aufweisen.

11. Der Verband nach Anspruch 1, wobei der Unterdruckverteiler ein beliebiges von einem Stoff, einem gewebten Material, einem Mull, einer vakuumausgebildeten Struktur oder einer geprägten Struktur ist.

12. Der Verband nach Anspruch 1, wobei der Instillationsverteiler eine Hydrophilie aufweist, die größer als eine Hydrophilie des Unterdruckverteilers ist.

13. Der Verband nach Anspruch 1, ferner aufweisend einen oder mehrere strahlenundurchlässige Streifen oder bedruckte Tintenstreifen auf dem Verband.

14. Der Verband nach Anspruch 1, wobei der Verbinder ein Ventil aufweist, das konfiguriert ist, um den ersten Kanal selektiv mit der Instillationspfadschicht und den zweiten Kanal mit der Unterdruckpfadschicht zu verbinden, um eine Richtung eines Fluidflusses in der inneren Kavität umzukehren.

15. Der Verband nach Anspruch 1, wobei der Verbinder konfiguriert ist, um mit einer Therapieeinheit fluidisch zu koppeln, wobei die Therapieeinheit aufweist:
eine Unterdruckpumpe, die konfiguriert ist, um mit dem ersten Kanal fluidisch zu koppeln, um einen Unterdruck in der inneren Kavität anzusaugen; und
eine Instillationspumpe, die konfiguriert ist, um mit dem zweiten Kanal fluidisch zu koppeln, um der inneren Kavität Instillationsfluid bereitzustellen.

## Revendications

1. Pansement pour une cavité interne, le pansement comprenant :
un élément de liaison ;
une couche de voie de pression négative accouplée à l'élément de liaison au niveau d'une première partie de la couche de voie de pression négative, dans lequel une partie de la couche de voie de pression négative est sensiblement libre ;
une couche de voie d'instillation accouplée à l'élément de liaison au niveau d'une première partie de la couche de voie d'instillation, dans lequel une partie de la couche de voie d'instillation est sensiblement libre ;
un collecteur de pression négative disposé à l'intérieur de la couche de voie de pression négative, dans lequel une première partie du collecteur de pression négative est accouplée de manière fluide à un premier canal de l'élément de liaison ; et
un collecteur d'instillation disposé dans la couche de voie d'instillation, dans lequel une partie du collecteur d'instillation est accouplée de manière fluide avec un second canal de l'élément de liaison ;
dans lequel la couche de voie de pression négative et la couche de voie d'instillation sont scellées le long d'une ou plusieurs parties d'un périmètre de la couche de voie de pression négative et d'un périmètre de la couche de voie d'instillation et sont conçues pour former ensemble un volume interne entre elles, dans lequel le volume interne est conçu pour recevoir un remplisseur d'espace ; et
dans lequel la couche de voie de pression négative, la couche de voie d'instillation et le remplisseur d'espace sont collectivement conçus pour être positionnés dans la cavité interne.

2. Pansement selon la revendication 1, dans lequel l'élément de liaison est conçu pour s'accoupler de manière fluide à un premier organe tubulaire au niveau du premier canal et à un second organe tubulaire au niveau du second canal, dans lequel le premier canal est accouplé de manière fluide à la première partie de la couche de voie de pression négative et le second canal est accouplé de manière fluide à la première partie de la couche de voie d'instillation, dans lequel le premier organe tubulaire, le premier canal et la couche de voie de pression négative sont conçus pour l'application d'une pression négative à la cavité interne et pour l'élimination de fluide de la cavité interne.

3. Pansement selon la revendication 2, dans lequel le second organe tubulaire, le second canal et la couche de voie d'instillation sont conçus pour une instillation de fluide dans la cavité interne.

4. Pansement selon la revendication 1, dans lequel le premier canal et le second canal s'étendent à travers l'élément de liaison.

5. Pansement selon la revendication 1, dans lequel la cavité interne est une cavité interne d'un sein d'un patient.

6. Pansement selon la revendication 1, dans lequel la couche de voie de pression négative et la couche de voie d'instillation sont des côtés opposés du pansement.

7. Pansement selon la revendication 1, dans lequel la couche de voie de pression négative comprend une couche de film supérieure et une couche de film inférieure avec le collecteur de pression négative disposé entre elles, dans lequel la couche de film supérieure et la couche de film inférieure sont des films de polyuréthane et un extérieur de la couche de film supérieure et de la couche de film inférieure de la couche de voie de pression négative est perforé ou fenêtré.

8. Pansement selon la revendication 1, dans lequel la couche de voie d'instillation comprend une couche de film supérieure et une couche de film inférieure avec le collecteur d'instillation disposé entre elles, dans lequel la couche de film supérieure et la couche de film inférieure de la couche de voie d'instillation sont des films de polyuréthane et un extérieur de la couche de film supérieure et de la couche de film inférieure de la couche de voie d'instillation est perforé ou fenêtré.

9. Pansement selon la revendication 1, dans lequel le remplisseur d'espace est un implant mammaire temporaire ou permanent ou une vessie ou un ballonnet gonflable.

10. Pansement selon la revendication 1, dans lequel le collecteur de pression négative et le collecteur d'instillation comprennent des bandes de mousse.

11. Pansement selon la revendication 1, dans lequel le collecteur de pression négative est l'un quelconque parmi un textile, un matériau en bande, une gaze, une structure formée sous vide ou une structure gaufrée.

12. Pansement selon la revendication 1, dans lequel le collecteur d'instillation a une hydrophilie supérieure à une hydrophilie du collecteur de pression négative.

13. Pansement selon la revendication 1, comprenant en outre un ou plusieurs rubans radio-opaques ou rubans d'encre imprimée sur le pansement.

14. Pansement selon la revendication 1, dans lequel l'élément de liaison comprend une vanne conçue pour lier entre eux sélectivement le premier canal avec la couche de voie d'instillation et le second canal avec la couche de voie de pression négative afin d'inverser une direction d'écoulement de fluide dans la cavité interne.

15. Pansement selon la revendication 1, dans lequel l'élément de liaison est conçu pour s'accoupler de manière fluide avec une unité de traitement, dans lequel l'unité de traitement comprend :
une pompe à pression négative conçue pour s'accoupler de manière fluide avec le premier canal afin d'aspirer une pression négative au niveau de la cavité interne ; et
une pompe d'instillation conçue pour s'accoupler de manière fluide au second canal afin de fournir un fluide d'instillation à la cavité interne.
